# EUROPEAN PATENT APPLICATION

(11) **EP 2 589 589 A1**
(43) Date of publication of application: **08.05.2013**
(21) Application number: 11800692.3
(22) Date of filing: 22.06.2011
(51) Int. Cl.: C07C 211/54, G03G 5/06

(54) **TRIPHENYLAMINE DERIVATIVE**

(30) Priority: 29.06.2010 JP 2010147368
(71) Applicant: Hodogaya Chemical Co., Ltd., Tokyo 104-0028 (JP)
(72) Inventor: NUMAZAWA, Shigetaka, Tsukuba-shi Ibaraki 305-0841 (JP); ABE, Katsumi, Koriyama-shi Fukushima 963-8802 (JP); IHARA, Kiyotaka, Shunan-shi Yamaguchi 746-0042 (JP); NAKAJIMA, Takehiro, Koriyama-shi Fukushima 963-8802 (JP); KOIKE, Makoto, Koriyama-shi Fukushima 963-8802 (JP)
(74) Representative: Müller-Boré & Partner Patentanwälte
(86) International application number: PCT/JP2011/064297
(87) International publication number: WO 2012/002227

(57) **Abstract**

[Problems] To provide a novel compound useful as an electric charge transporting agent having a high carrier mobility, and making it possible not only to stably form a photosensitive layer without precipitating crystals or developing pinholes when the photosensitive layer is being formed but also to prepare an organic photosensitive material for electrophotography featuring a high sensitivity and a low residual potential.

[Means for Solution] The compound is represented by the following general formula (1);

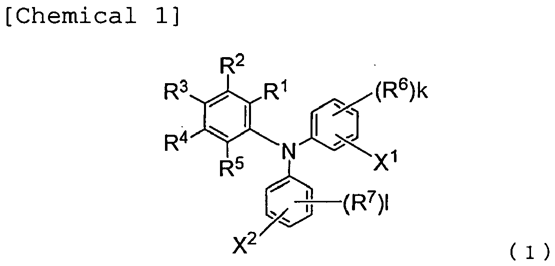

In the general formula (1), R¹ to R⁷ are, for example, alkyl groups, X¹ is, for example, -CH=CH-CH=CH₂, X² is, for example, -CH=CH₂, and k and 1 are integers of 0 to 4.

## Description

### Technical Field:

This invention relates to triphenylamine derivatives having a high carrier mobility, are useful as an electric charge transporting agent which are used for a photosensitive material for electrophotography.

### Background Art:

Amorphous silicon, amorphous selenium, cadmium sulfide, zinc oxide and the like have heretofore been known as inorganic photoconductive materials. Inorganic photosensitive materials formed by using such photoconductive materials have been widely used in the field of electrophotography. However, selenium and cadmium sulfide are toxic substances and must be recovered. Besides, selenium undergoes crystallization when heated and, therefore, has poor heat resistance, while the cadmium sulfide and zinc oxide have poor resistance against the humidity, the zinc oxide, further, has small endurance against the printing. In recent years, therefore, it is becoming a main stream to use an organic photosensitive material comprising an electrically conducting substrate on which is formed an organic photosensitive layer that contains an electric charge generating agent and an electric charge transporting agent as photoconductive materials.
As such organic photosensitive materials, there have been known those of the single-layer type in which a photosensitive layer formed on the electrically conducting substrate contains a charge generating agent and a charge transporting agent that are dispersed in a resin binder, and those of the lamination type in which the photosensitive layer comprises a charge generating layer of a charge generating agent that is dispersed in a resin binder and a charge transporting layer of a charge transporting agent that is dispersed in a resin binder. The organic photosensitive materials of both types have advantages such as light weight compared to the inorganic photosensitive materials, making it easy to form the photosensitive layer and, further, little affecting the environment.

In the above organic photosensitive material for electrophotography, the electric charge transporting agent must satisfy such requirements as efficiently receiving the carriers (positive electric charge or negative electric charge) generated by the electric charge generating agent upon the irradiation with light when an electric field is being applied, quickly moving the carriers in the photosensitive layer, and quickly extinguishing the electric charge in the surface of the photosensitive layer. The moving speed of the carrier in a unit electric field is called carrier mobility, and a high carrier mobility means that the carrier is allowed to quickly move in the photosensitive layer (or in the charge transporting layer) . The carrier mobility is specific to a compound used as the charge transporting agent. Therefore, the charge transporting agent must be a compound that has a high carrier mobility.

Further, the charge transporting agent and the charge generating agent are dissolved in an organic solvent together with the resin binder, and are applied and dried (removal of organic solvent) to form a photosensitive layer. Therefore, the charge transporting agent must also satisfy the requirement of forming a homogeneous photosensitive layer without precipitating crystals or without generating pinholes. If the photosensitive layer contains portions where the crystals are precipitated or contains pinholes, dielectric breakdown occurs in such portions giving rise to the occurrence of defects in the image when the image is formed by the electrophotography.

As described above, a variety of properties are required for the charge transporting agent and, therefore, a variety of compounds have heretofore been proposed as charge transporting agents (see patent documents 1 to 14).

### Prior Art Documents:

### Patent Documents:

Patent document 1: JP-B-58-32372
Patent document 2: JP-A-1-142642
Patent document 3: JP-A-5-088389
Patent document 4: JP-B-7-021646
Patent document 5: JP-B-5-019701
Patent document 6: JP-B-55-042380
Patent document 7: JP-A-57-101844
Patent document 8: JP-A-54-150128
Patent document 9: JP-A-61-023154
Patent document 10: JP-A-9-292723
Patent document 11: JP-A-60-340999
Patent document 12: JP-A-61-023154
Patent document 13: JP-B-58-032372
Patent document 14: U.S. Patent No. 3873312
Patent document 15: JP-A-8-211636

### Outline of the Invention:

### Problems that the Invention is to Solve:

When photosensitive layers are formed by using the charge generating layers and the charge transporting layers in combination, few of many compounds proposed in the above patent documents as charge transporting agents satisfy the properties and conditions really needed for the photosensitive materials. Namely, various problems remain, such as that crystals precipitate after a film is formed, that a sufficiently large surface potential is not maintained when dark even if a film is formed, and that the surface potential cannot be attenuated to a sufficient degree after irradiated with light (low sensitivity, high residual potential, etc.).

It is, therefore, an object of the present invention to provide a novel compound useful as an electric charge transporting agent having a high carrier mobility, and making it possible not only to stably form a photosensitive layer without precipitating crystals or developing pinholes when the photosensitive layer is being formed but also to prepare an organic photosensitive material for electrophotography featuring a high sensitivity and a low residual potential.
Another object of the present invention is to provide an electric charge transporting agent comprising the above compound, and an organic photosensitive material for electrophotography that contains the above electric charge transporting agent in the photosensitive layer.

### Means for Solving the Problems:

According to the present invention, there is provided a triphenylamine derivative represented by the following general formula (1). 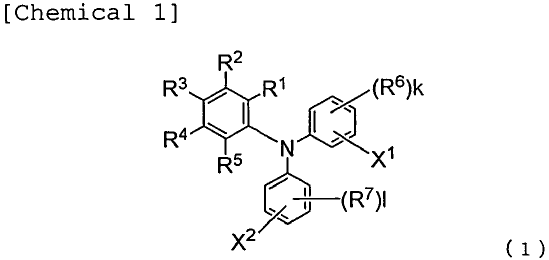 wherein,
k and 1 are respectively integers of 0 to 4,
R¹ to R⁵ may be the same or different, and are any groups selected from group consisting of hydrogen atom, deuterium atom, halogen atom, alkyl group having 1 to 6 carbon atoms, cycloalkyl group having 5 to 10 carbon atoms, alkenyl group having 2 to 6 carbon atoms, alkoxy group having 1 to 6 carbon atoms, cycloalkyloxy group having 5 to 10 carbon atoms, aromatic hydrocarbon group, aromatic heterocyclic ring group, condensed polycyclic aromatic group, and aryloxy group; or
among R¹ to R⁵, the two groups present at neighboring positions are bonded together to form a ring, and the other three groups are any groups selected from the above group;
R⁶ and R⁷ may be the same or different, and are the groups selected from the group consisting of deuterium atom; halogen atom; alkyl group having 1 to 6 carbon atoms; alkoxy group having 1 to 6 carbon atoms; aromatic hydrocarbon group; aromatic heterocyclic ring group; condensed polycyclic aromatic group; and di-substituted amino group having, as a substituent, alkyl group having 1 to 6 carbon atoms, alkenyl group having 2 to 6 carbon atoms, aralkyl group, aromatic hydrocarbon group or aromatic heterocyclic ring group;,
if R⁶ and R⁷ are present in plural numbers, pluralities of R⁶ and R⁷ may be the same or different,
X¹ is a monovalent group represented by the following general formula (1a);

   -CR⁸=CR⁹-CR¹⁰=CR¹¹R¹² (1a)

   wherein,
   R⁸ to R¹² may be the same or different and are hydrogen atoms, deuterium atoms, alkyl groups having 1 to 6 carbon atoms, alkoxy groups having 1 to 6 carbon atoms, aromatic hydrocarbon groups, aromatic heterocyclic ring groups or condensed polycyclic aromatic groups, wherein R¹¹ and R¹² together may form a ring and if R¹¹ is a hydrogen atom, deuterium atom or alkyl group, R¹² is an aromatic hydrocarbon group, aromatic heterocyclic ring group or condensed polycyclic aromatic group, and
X² is a monovalent group represented by the following general formula (1b);

   - (CR¹³=CR¹⁴)ₙ-CR¹⁵=CR¹⁶R¹⁷ (1b)

   wherein,
   n is 0 or 1,
   R¹³ to R¹⁷ may be the same or different and are hydrogen atoms, deuterium atoms, alkyl groups having 1 to 6 carbon atoms, alkoxy groups having 1 to 6 carbon atoms, aromatic hydrocarbon groups, aromatic heterocyclic ring groups or condensed polycyclic aromatic groups, wherein R¹⁶ and R¹⁷ together may form a ring and if R¹⁶ is a hydrogen atom, deuterium atom or alkyl group, R¹⁷ is an aromatic hydrocarbon group, aromatic heterocyclic ring group or condensed polycyclic aromatic group.

According to the present invention, there is, further, provided an electric charge transporting agent comprising the above triphenylamine derivative.

According to the present invention, there is, further, provided an organic photosensitive material for electrophotography having an organic photosensitive layer formed on an electrically conducting substrate, the organic photosensitive layer containing the above triphenylamine derivative as a charge transporting agent.

In the organic photosensitive material for electrophotography of the present invention, it is desired that:
(1) The organic photosensitive layer is a lamination type photosensitive layer comprising a charge generating layer in which a charge generating agent is dispersed in a resin binder and a charge transporting layer in which the charge transporting agent is dispersed in a resin binder; and
(2) The organic photosensitive layer is a single-layer type photosensitive layer in which the charge generating agent and the charge transporting agent are dispersed in a resin binder.

### Effects of the Invention:

The triphenylamine derivative of the invention represented by the above general formula (1) is a novel compound having a high carrier mobility, and is very useful as a charge transporting agent which is used for the production of an organic photosensitive material for electrophotography.
Further, the organic photosensitive material that contains the above triphenylamine derivative as a charge transporting agent does not precipitate crystals and does not develop pinholes when it is used for forming a photosensitive layer (film), and features a high sensitivity, a low residual potential, a small fluctuation in the surface potential, a small decrease in the sensitivity and a small accumulation of residual potential even after the images are repetitively formed by electrophotography, offering excellent durability.

### Brief Description of the Drawings:

[Fig. 1] is an IR spectrum of a compound (Example compound No. 1) of Example 1 of the invention.
[Fig. 2] is an IR spectrum of a compound (Example compound No. 10) of Example 3 of the invention.

### Modes for Carrying Out the Invention:

### <Triphenylamine derivatives>

The triphenylamine derivative of the invention is represented by the following general formula (1).

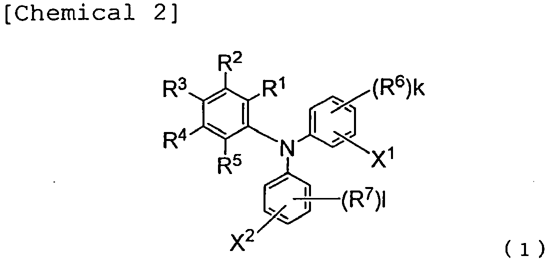

In the above general formula (1), k represents a number of the groups R⁶ and is an integer of 0 to 4, and 1 represents a number of the groups R⁷ and is an integer of 0 to 4.
The groups R¹ to R⁷ and the groups X¹ and X² are as described below.

### (Groups R¹ to R⁵)

The groups R¹ to R⁵, respectively, may be the same or different, and are any groups selected from group consisting of hydrogen atom, deuterium atom, halogen atom, alkyl group, cycloalkyl group, alkenyl group, alkoxy group, aromatic hydrocarbon group, aromatic heterocyclic ring group, condensed polycyclic aromatic group, and aryloxy group, or, among R¹ to R⁵, the two groups present at neighboring positions are bonded together to form a ring, and the other three groups are any groups selected from the above group.

Among the groups described in the above group, examples of the halogen atom include fluorine atom, chlorine atom, bromine atom and iodine atom.

The alkyl group is a lower alkyl group having carbon atoms in a number in a range of 1 to 6. The lower alkyl group may be in the form of a straight chain or a branch.
Concrete examples of the alkyl group include methyl group, ethyl group, n-propyl group, isopropyl group, n-butyl group, isobutyl group, tert-butyl group, n-pentyl group, isopentyl group, neopentyl group and n-hexyl group.

The cycloalkyl group has carbon atoms in a number in a range of 5 to 10. Examples of the cycloalkyl group include cyclopentyl group, cyclohexyl group, 1-adamantyl group and 2-adamantyl group.

The alkenyl group has carbon atoms in a number in a range of 2 to 6. Examples of the alkenyl group include vinyl group, allyl group, isopropenyl group and 2-butenyl group.

The alkoxy group has carbon atoms in a number in a range of 1 to 6. Examples of the alkoxy group include methyloxy group, ethyloxy group, n-propyloxy group, isopropyloxy group, n-butyloxy group, tert-butyloxy group and n-pentyloxy group.

The cycloalkoxy group has carbon atoms in a number in a range of 5 to 10. Examples of the cycloalkoxy group include n-hexyloxy group, cyclopentyloxy group, cyclohexyloxy group, cycloheptyloxy group, cyclooctyloxy group, 1-adamantyloxy group and 2-adamantyloxy group.

Examples of the aromatic hydrocarbon group include phenyl group, biphenylyl group and terphenylyl group.

Examples of the condensed polycyclic aromatic group include naphtyl group, anthryl group, phenanthryl group, fluorenyl group, indenyl group, pyrenyl group, perylenyl group, fluoranthenyl group and triphenylenyl group.

Examples of the aromatic heterocyclic ring group include pyridyl group, furanyl group, pyranyl group, thiophenyl group, quinolyl group, isoquinolyl group, benzofuranyl group, benzothiophenyl group, indolyl group, carbazolyl group, benzoxazolyl group, benzothiazolyl group, quinoxalyl group, benzoimidazolyl group, pyrazolyl group, dibenzofuranyl group, dibenzothiophenyl group, carbolinyl group, phenoxy group, tolyloxy group, biphenylyloxy group, terphenylyloxy group, naphthyloxy group, anthryloxy group, phenanthryloxy group, fluorenyloxy group, indenyloxy group, pyrenyloxy group and perylenyloxy group.

The above groups R¹ to R⁵ may, further, have substituents. As the substituents, the following groups can be exemplified on condition that they satisfy predetermined numbers of carbon atoms.
Deuterium atom;
Trifluoromethyl group;
Cyano group;
Nitro group;
Halogen atom such as fluorine atom, chlorine atom,
bromine atom or iodine atom;
Alkoxy group having 1 to 6 carbon atoms, such as methoxy
group, ethoxy group or propyloxy group;
Alkenyl group such as allyl group;
Aryloxy group such as phenoxy group or tolyloxy group;
Arylalkoxy group such as benzyloxy group or phenetyloxy
group;
Aromatic hydrocarbon group such as phenyl group,
biphenylyl group or terphenylyl group;
Condensed polycyclic aromatic group such as naphthyl group, anthracenyl group, phenanthryl group, fluolenyl group, indenyl group, pyrenyl group, perylenyl group, fluoranthenyl group or triphenylenyl group;
Aromatic heterocyclic ring group such as pyridyl group, furanyl group, pyranyl group, thienyl group, furyl group, pyrolyl group, thiophenyl group, quinolyl group, isoquinolyl group, benzofuranyl group, benzothiophenyl group, indolyl group, carbazolyl group, benzoxazolyl group, benzothioazolyl group, quinoxalyl group, benzoimidazolyl group, pyrazolyl group, dibenzofuranyl group, dibenzothiophenyl group or carbolinyl group.

Among the above groups R¹ to R⁵, further, the two neighboring groups (e.g., group R³ and group R⁹ or group R⁴ and group R⁵) may be bonded together to form a ring. In this case, the other three groups are any groups exemplified above.
As the ring formed by such two groups bonded together, there can be exemplified an aliphatic hydrocarbon ring (cyclopentane ring, cyclohexane ring or cycloheptane ring), aromatic hydrocarbon ring such as benzene ring, heterocyclic ring such as pyrrole ring, and a polycyclic condensed ring formed by the condensation of the above rings (e. g. , indane ring formed by the condensation of benzene ring and cyclopentane ring, or indole ring formed by the condensation of benzene ring and pyrrole ring). These rings may have a substituent mentioned above.

### (Groups R⁶ and R⁷)

The above groups R⁶ and R⁷ may be the same or different from each other, and are the groups selected from a group constitution of deuterium atom, halogen atom, alkyl group having 1 to 6 carbon atoms, alkoxy group having 1 to 6 carbon atoms, aromatic hydrocarbon group, aromatic heterocyclic ring group, condensed polycyclic aromatic group, and di-substituted amino group.
If the groups R⁶ and R⁷ are present in plural numbers, pluralities of the groups R⁶ and R⁷ may be the same or different from each other.

Among the above groups, halogen atom, alkyl group, alkoxy group, aromatic hydrocarbon group, aromatic heterocyclic ring group and condensed polycyclic aromatic group may be the same groups as those exemplified for the above groups R¹ to R⁵.

The di-substituted amino group has two substituents bonded to nitrogen atoms thereof, the substituents being selected from the group consisting of alkyl group having 1 to 6 carbon atoms (which may be a straight chain or in a branched form), alkenyl group having 2 to 6 carbon atoms (which may be a straight chain or in a branched form, e.g., aryl group), aralkyl group (e.g., benzyl group or phenetyl group), aromatic hydrocarbon group and aromatic heterocyclic ring group. Among them, alkyl group, alkenyl group, aromatic hydrocarbon group and aromatic heterocyclic ring group may be those described above.

Examples of the di-substituted amino group having the above substituents include dialkylamino groups such as dimethylamino group and diethylamino group; diarylamino groups such as diphenylamino group and dinaphthylamino group; diaralkylamino groups such as dibenzylamino group and diphenetylamino group; diheteroarylamino groups such as dipyridylamino group and dithienylamino group; and dialkenylamino groups such as diallylamino group.

The above alkyl group, alkoxy group, aromatic hydrocarbon group, aromatic heterocyclic ring group and condensed polycyclic aromatic group may have a substituent, and the substituents possessed by the above di-substituted amino group may have another substituent.
As such substituents, there can be exemplified the same substituents as those exemplified for the groups R¹ to R⁵.

### (Group X¹)

The group X¹ in the general formula (1) is a monovalent group represented by the following general formula (1a).

-CR⁸=CR⁹-CR¹⁰=CR¹¹R¹² (1a)

In the general formula (1a), R⁸ to R¹² may be the same or different and are hydrogen atoms, deuterium atoms, straight-chain or branched alkyl groups having 1 to 6 carbon atoms, straight-chain or branched alkoxy groups having 1 to 6 carbon atoms, aromatic hydrocarbon groups, aromatic heterocyclic ring groups or condensed polycyclic aromatic groups. Concrete examples of the groups R⁸ to R¹² may be the same groups as those exemplified above for the groups R¹ to R⁵. Further, the groups R⁸ to R¹², too, may have the same substituents (excluding, however, halogen) as those exemplified for the groups R¹ to R⁵

Among the above groups R⁸ to R¹², further, if R¹¹ is a hydrogen atom or alkyl group, R¹² is an aromatic hydrocarbon group, aromatic heterocyclic ring group or condensed polycyclic aromatic group.

Further, the groups R¹¹ and R¹² together may form a ring. For example, R¹¹ and R¹² may be directly bonded together or may be bonded together via methylene group, ethylene group, carbonyl group, vinylidene group or ethylenylene group to form a hydrocarbon ring or a heterocyclic ring that includes oxygen atom, sulfur atom or nitrogen atom.

### (Group X²)

The group X² in the general formula (1) is a monovalent group represented by the following general formula (1b).

- (CR¹³=CR¹⁴)ₙ-CR¹⁵=CR¹⁶R¹⁷ (1b)

In the general formula (1b), n is a number of - (CR¹³=CR¹⁴) and is 0 or 1.

In the above formula, R¹³ to R¹⁷ may be the same or different and are hydrogen atoms, deuterium atoms, straight-chain or branched alkyl groups having 1 to 6 carbon atoms, straight-chain or branched alkoxy groups having 1 to 6 carbon atoms, aromatic hydrocarbon groups, aromatic heterocyclic ring groups or condensed polycyclic aromatic groups.
Concrete examples of the groups R¹³ to R¹⁷ may be the same groups as those exemplified for the groups R¹ to R⁵
As the alkyl group having 1 to 6 carbon atoms, there can be exemplified methyl group, ethyl group, propyl group, butyl group, hexyl group, tert-butyl group and isopropyl group.
As the alkoxy group having 1 to 6 carbon atoms, there can be exemplified methoxy group, ethoxy group and propyloxy group.
As the aromatic hyrocarbon group, there can be exemplified phenyl group.
As the aromatic heterocyclic ring group, there can be exemplified pyridyl group, pyrrolyl group, thienyl group, furyl group, carbazolyl group and pyrrolinyl group.
As the condensed polycyclic aromatic group, there can be exemplified naphthyl group, anthracenyl group and pyrenyl group.

Further, the groups R¹³ to R¹⁷, too, may have the same substituents (excluding, however, halogen) as those exemplified above for the groups R¹ to R⁵

Among the above groups R¹³ to R¹⁷, further, if R¹⁶ is a hydrogen atom or alkyl group, R¹⁷ is an aromatic hydrocarbon group, aromatic heterocyclic ring group or condensed polycyclic aromatic group.

Further, among these groups, R¹⁶ and R¹⁷ together may form a ring like the above groups R¹¹ and R¹². For example, R¹⁶ and R¹⁷ may be directly bonded together or may be bonded together via methylene group, ethylene group, carbonyl group, vinylidene group or ethylenylene group to form a hydrocarbon ring or a heterocyclic ring that includes oxygen atom, sulfur atom or nitrogen atom.

In the triphenylamine derivative of the invention mentioned above, it is desired that among R¹ to R⁵, the two groups at neighboring positions are bonded together to form a ring from the standpoint of a property as the charge transporting agent. In this case, the other three groups are any of the groups selected from the group mentioned above or are, concretely, any of hydrogen atom, deuterium atom, halogen atom, alkyl group having 1 to 6 carbon atoms, cycloalkyl group having 5 to 10 carbon atoms, alkenyl groups having 2 to 6 carbon atoms, alkoxy group having 1 to 6 carbon atoms, cyloalkyloxy group having 5 to 10 carbon atoms, aromatic hydrocarbon group, aromatic heterocyclic ring group, condensed polycyclic aromatic group or aryloxy group.

As the ring formed by the two groups at neighboring positions that are bonded together among R¹ to R⁵, there can be exemplified aliphatic hydrocarbon ring (specifically, five-membered ring or six-membered ring) and heterocyclic ring (e.g., pyrrole ring). These rings may have a polycyclic structure condensed with an aromatic ring such as benzene ring, and to which, further, the above-mentioned various substituents may be bonded.

The triphenylamine derivative of the present invention having the most desired structure is the one in which the above-mentioned ring is the aliphatic five-membered ring or the aliphatic six-membered ring and, concretely, is the one represented by the following general formula(2) or (3).

general formula (2); 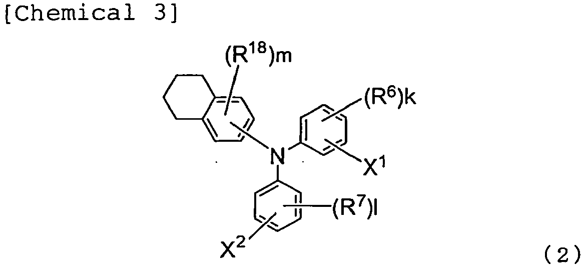 general formula (3); 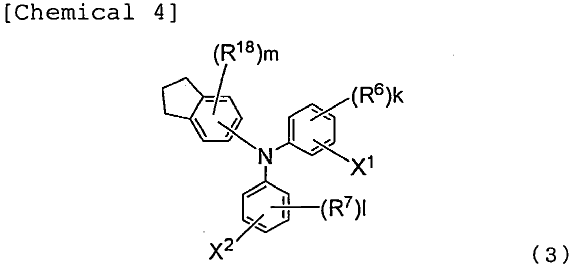 In the general formulas (2) and (3),
k, 1, R⁶, R⁷, X¹ and X² are respectively as defined in the above general formula (1),
m is an integer of 0 to 3,
R¹⁸ is any group selected from the group consisting of deuterium atom, halogen atom, alkyl group, cycloalkyl group, alkenyl group, alkoxy group, aromatic hydrocarbon group, aromatic heterocyclic ring group, condensed polycyclic aromatic group and aryloxy group. As the group R¹⁸, there can be concretely exemplified the same groups as those exemplified above for the groups R¹ to R⁵. The group R¹⁸ may have the same substituents as those for the groups R¹ to R⁵ mentioned above. If there are a plurality of groups R¹⁸, the plurality of the groups R¹⁸ may be the same or different.

Further, the triphenylamine derivative of the above general formula (2) preferably has a structure in which the groups R³ and R⁴ in the general formula (1) are bonded together to form a ring (six-membered ring), and is, concretely, a compound having the following general formula (2-1); 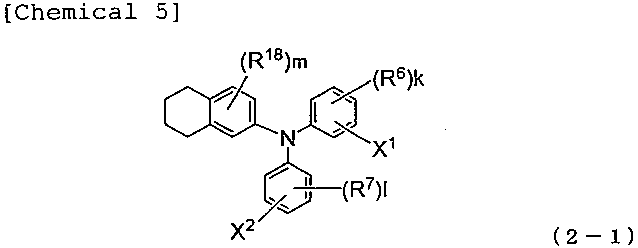 wherein k, 1, m, R⁶, R⁷, R¹⁸, X¹ and X² are as
defined in the above general formula (2).

Further, of the triphenylamine derivatives represented by the above general formula (2-1), a preferred compound is the one having a structure in which the groups X¹ and X² in the general formula (1) are bonded to para-positions with respect to the nitrogen atom, and, concretely, is a compound represented by the following general formula (2-2); 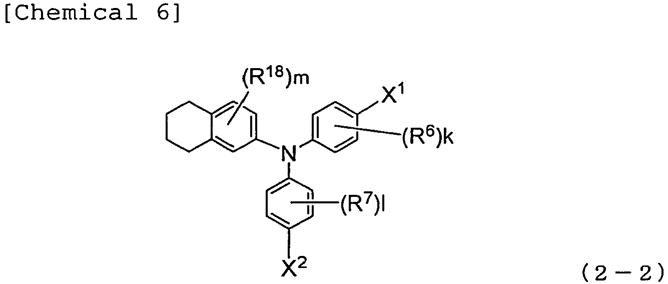 wherein k, l, m, R⁶, R⁷, R¹⁸, X¹ and X² are respectively as defined in the above general formula (2-1).

The triphenylamine derivatives represented by the above general formula (2-2) include those of the two kinds represented by the following general formulas (2-3) and (2-3'). Triphenylamine derivatives of the general formula (2-3); 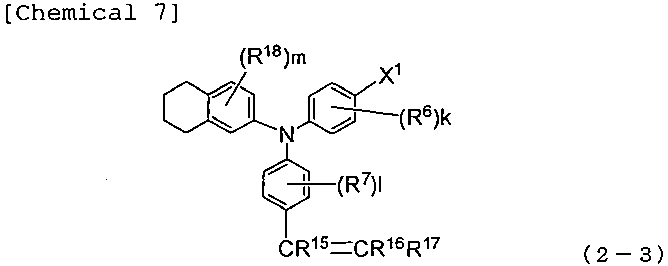 Tirphenylamine derivatives of the general formula (2-3'); 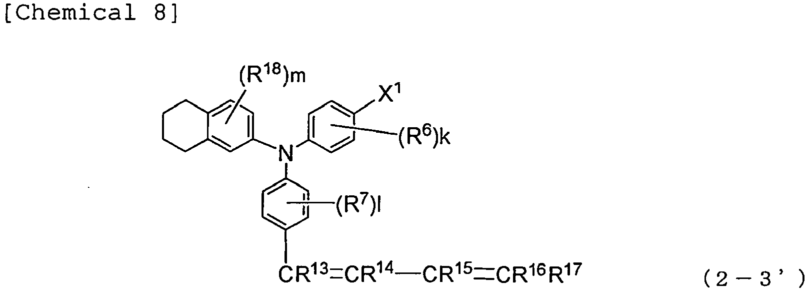 In the above general formulas (2-3) and (2-3'), k, l, m, R⁶, R⁷, R¹⁸ and X¹ are respectively as defined in the above general formula (2-2), and R¹³ to R¹⁷ are respectively as defined in the above general formula (1b).
Namely, in the case of the triphenylamine derivative represented by the general formula (2-3), n of the group X² in the general formula (1) is 0 while in the case of the general formula (2-3'), n of the group X² is 1.

Preferably, further, the triphenylamine derivative of the above general formula (3) has a structure in which the groups R³ and R⁴ in the general formula (1) are bonded together to form a ring (five-membered ring), and is, concretely, a compound having the following general formula (3-1); 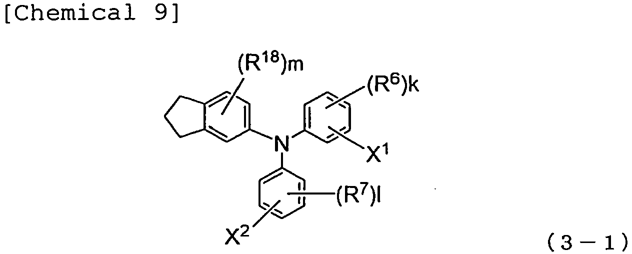 wherein k, l, m, R⁶, R⁷, R¹⁸, X¹ and X² are respectively as defined in the above general formula (3).

Of the triphenylamine derivatives of the present invention, concrete examples of the preferred compounds are as described below.

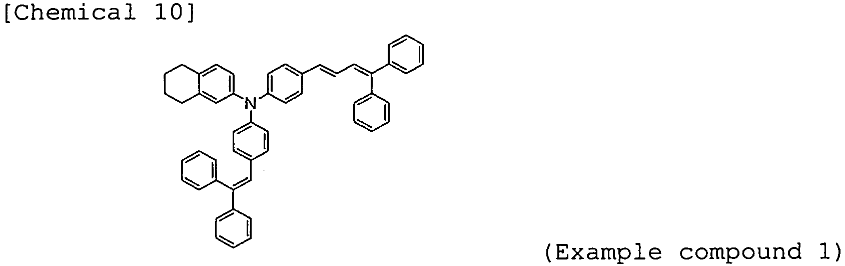

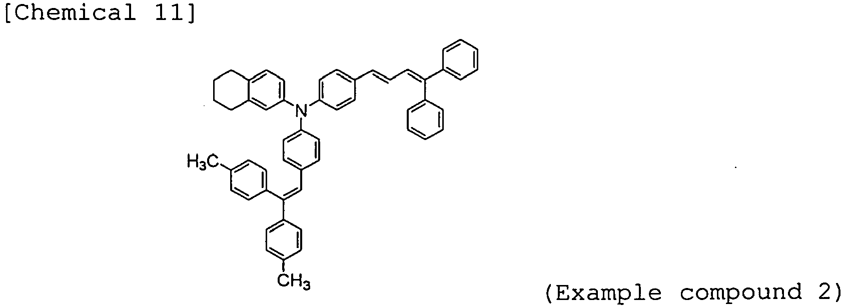

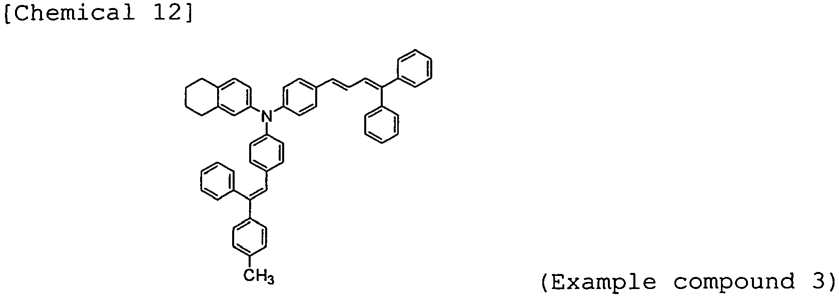

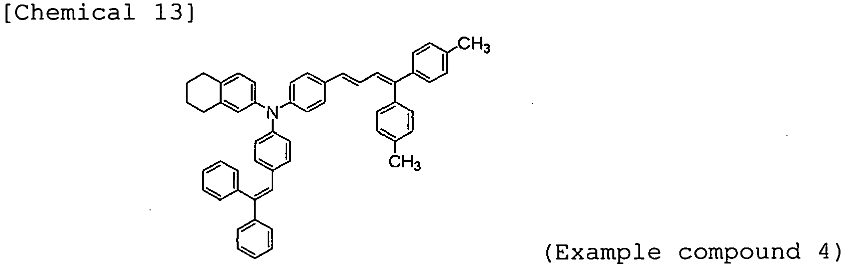

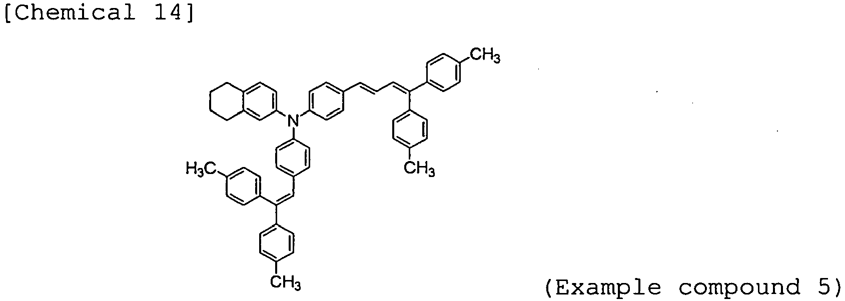

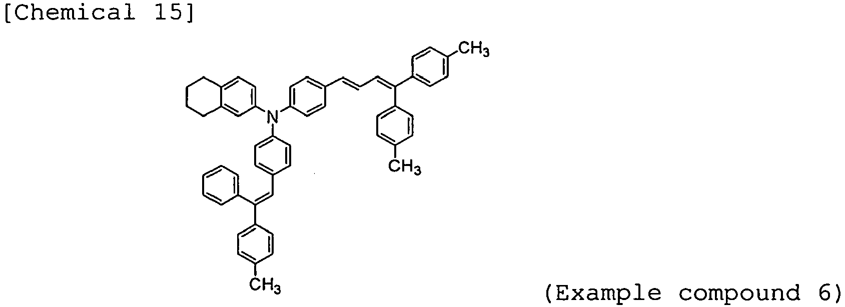

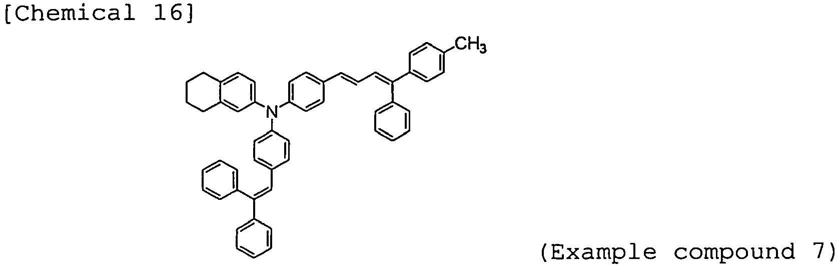

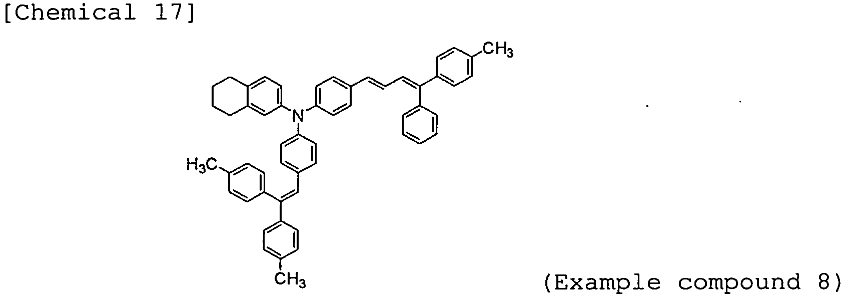

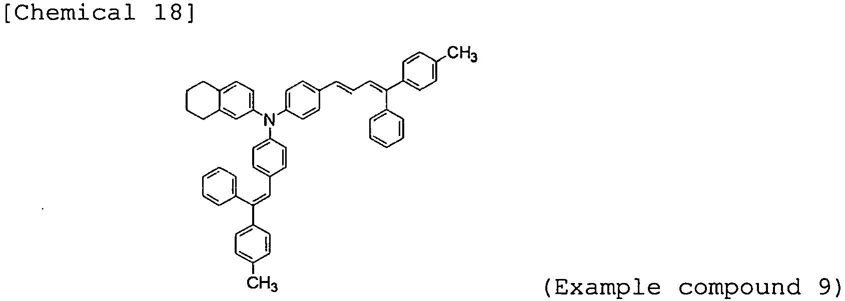

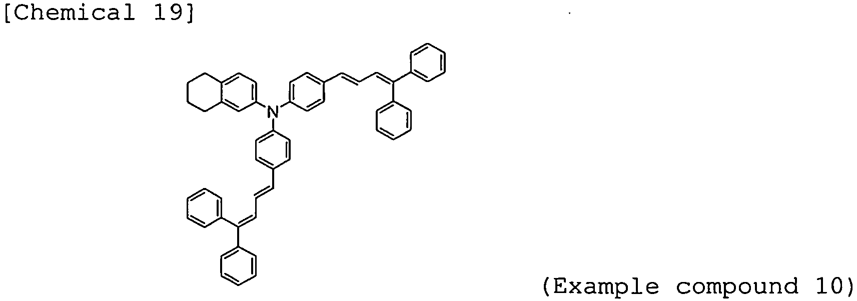

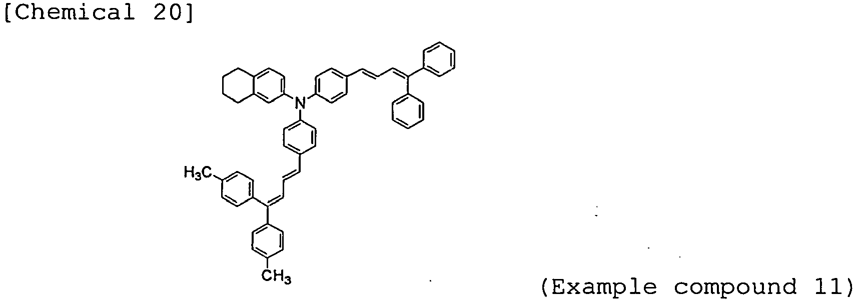

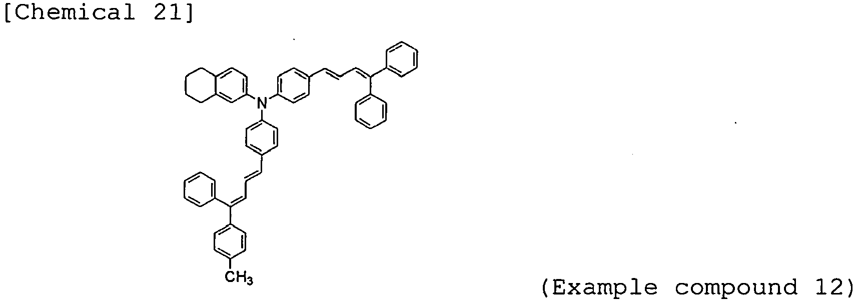

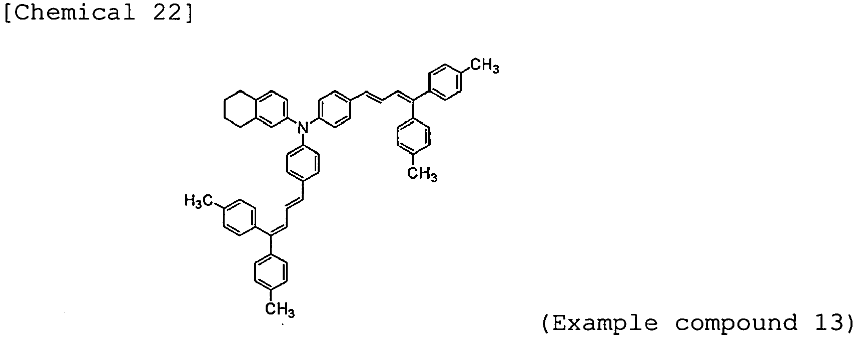

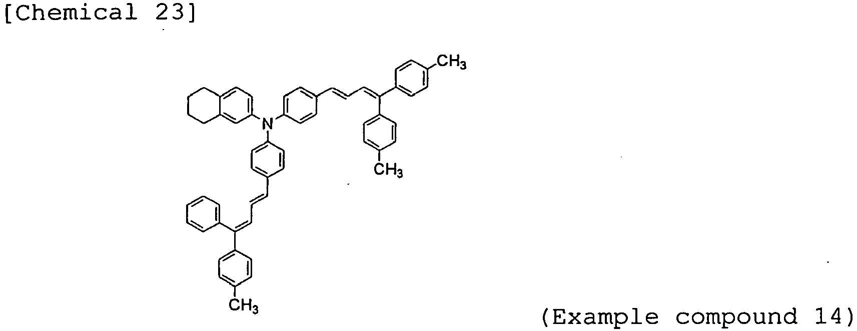

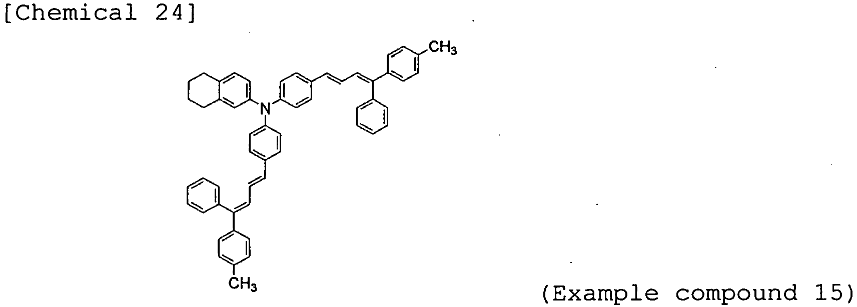

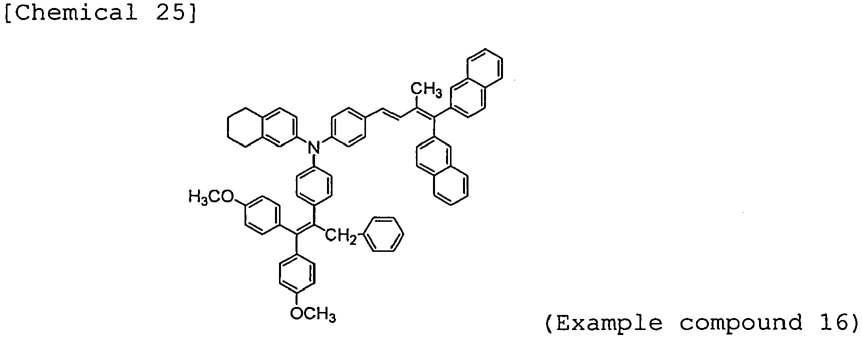

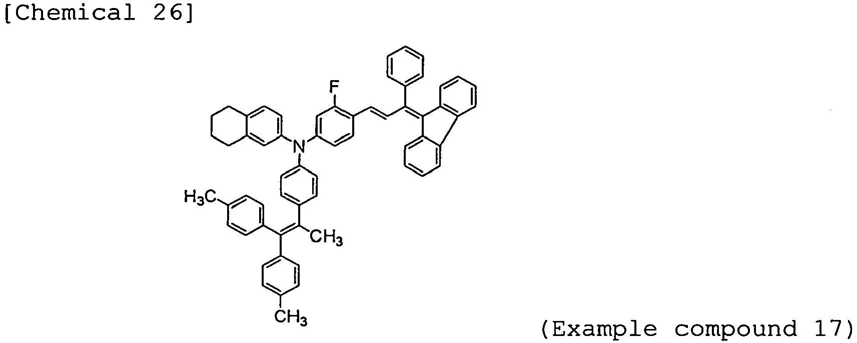

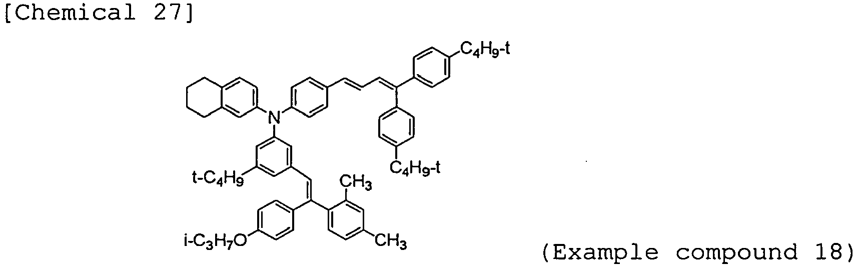

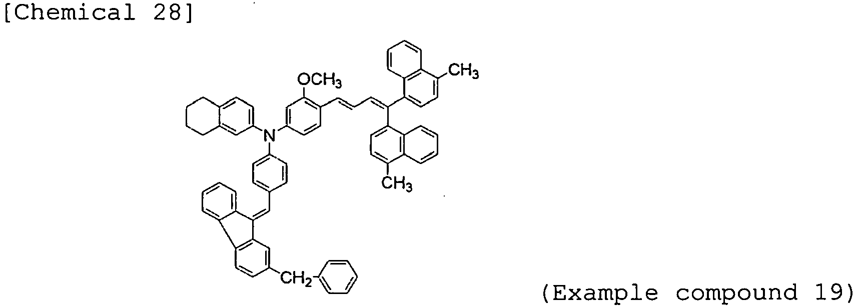

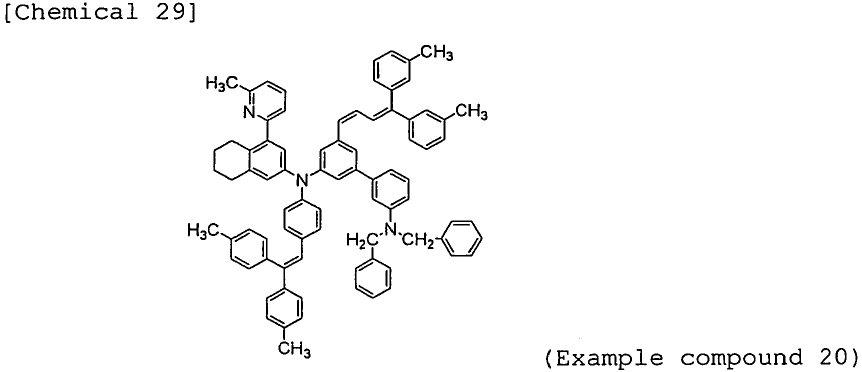

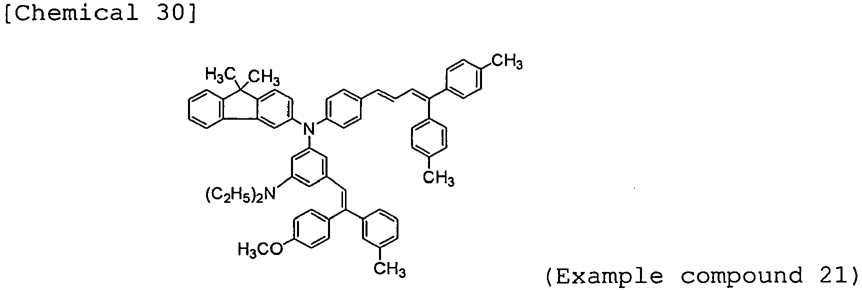

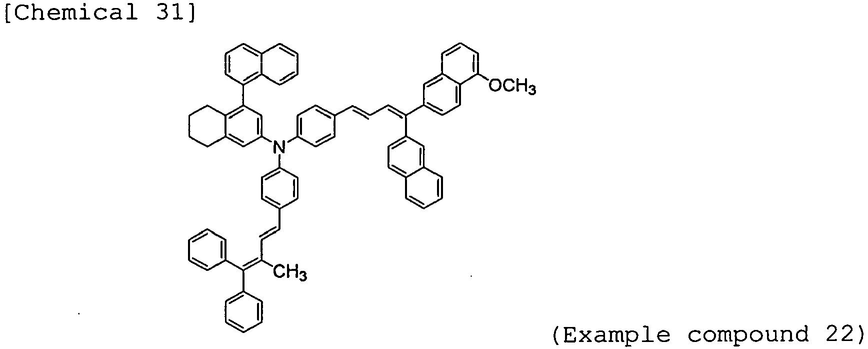

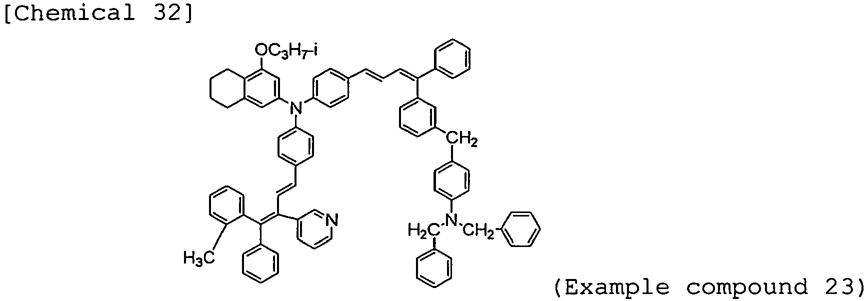

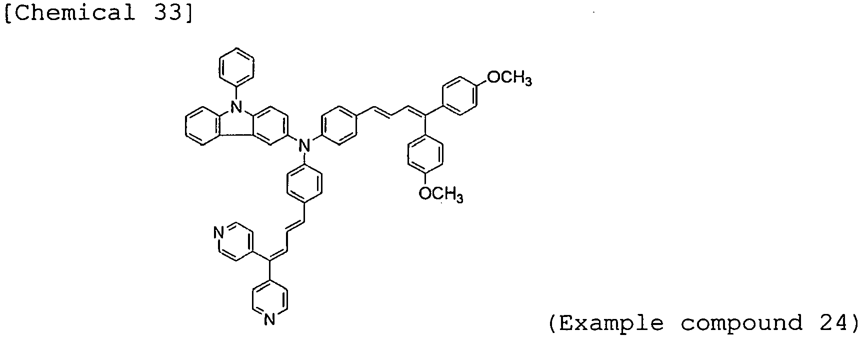

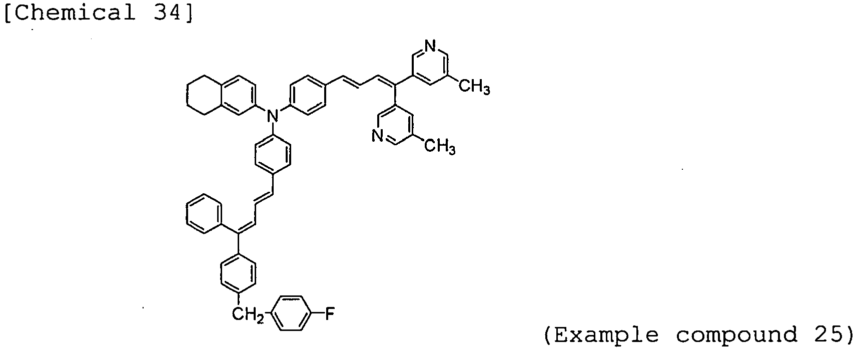

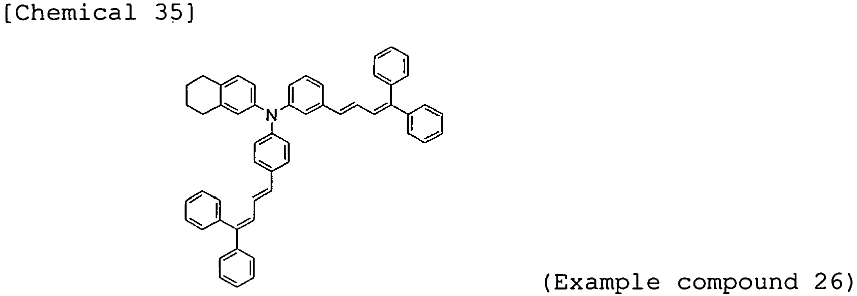

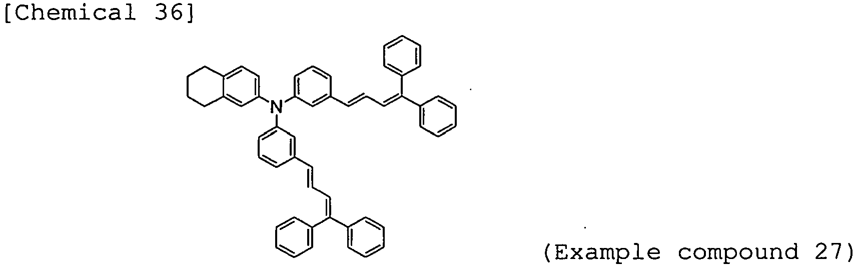

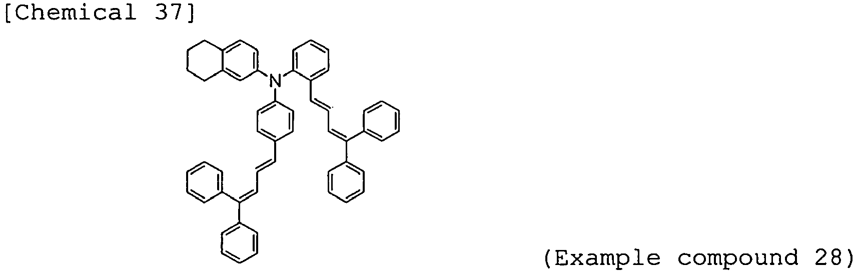

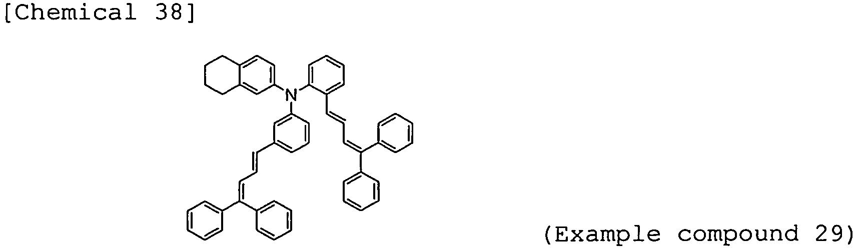

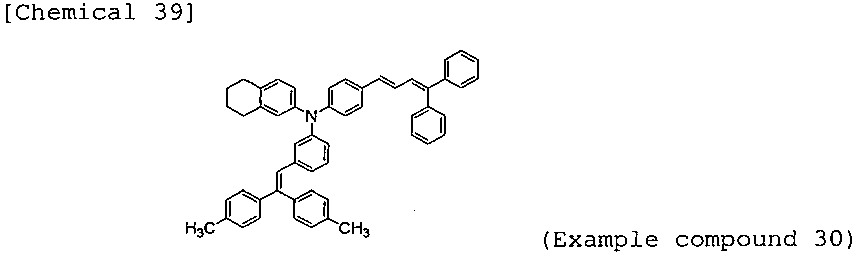

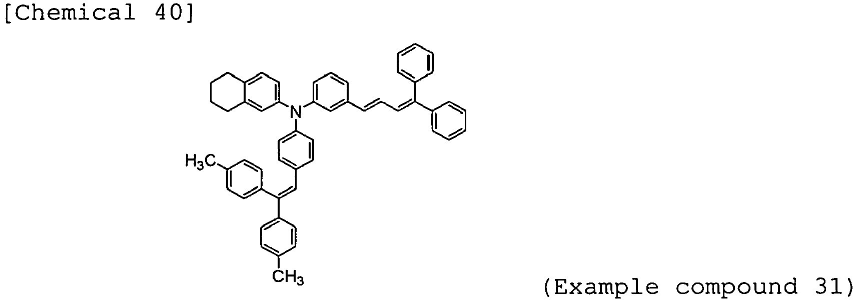

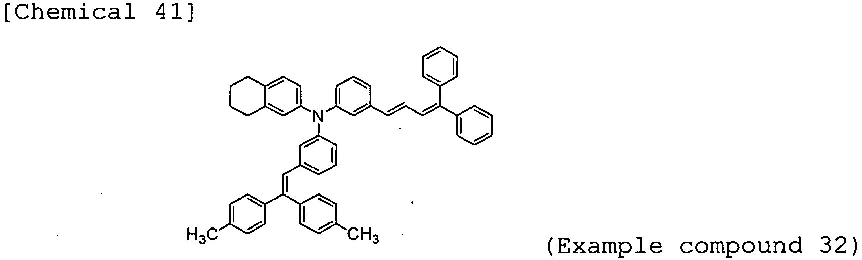

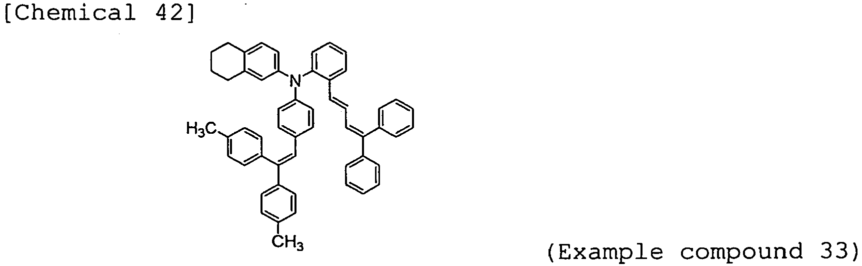

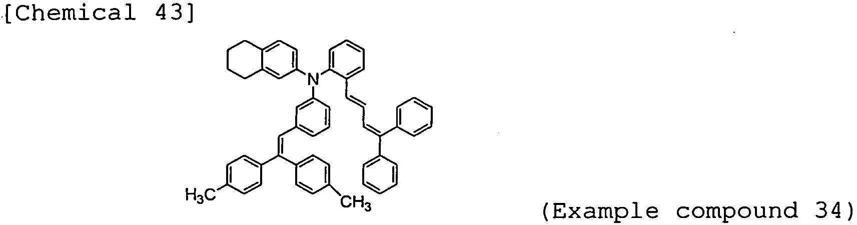

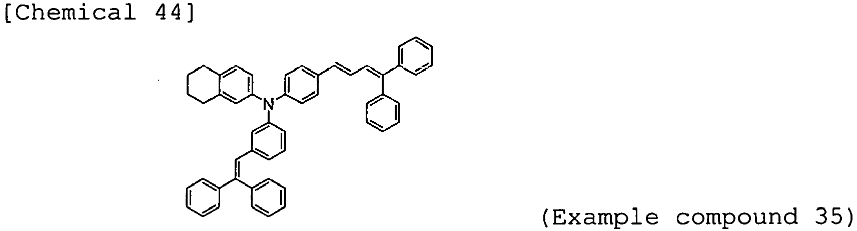

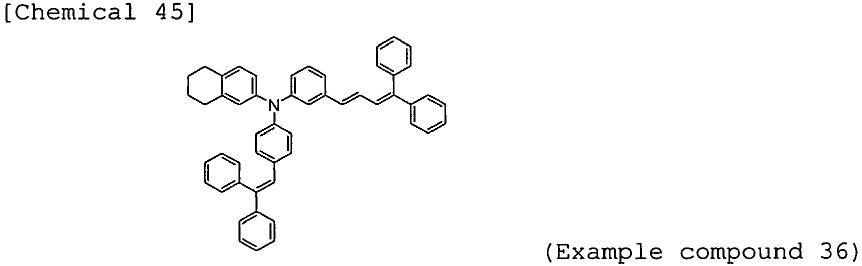

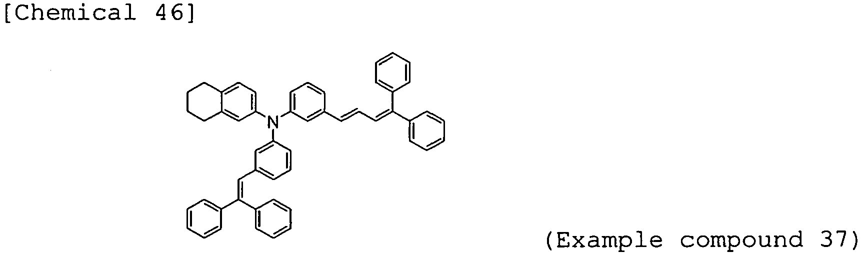

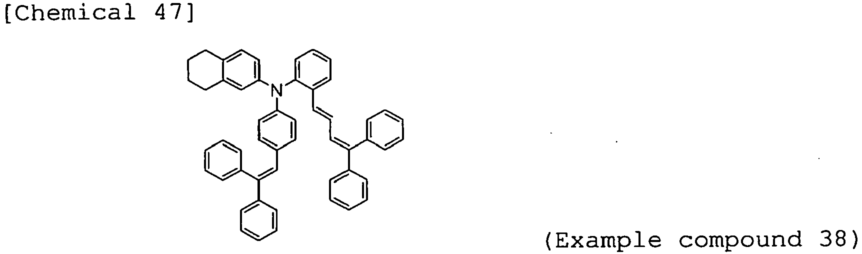

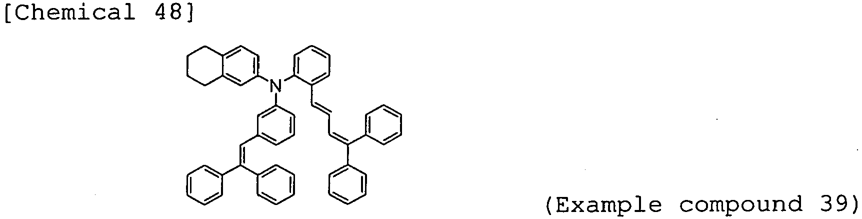

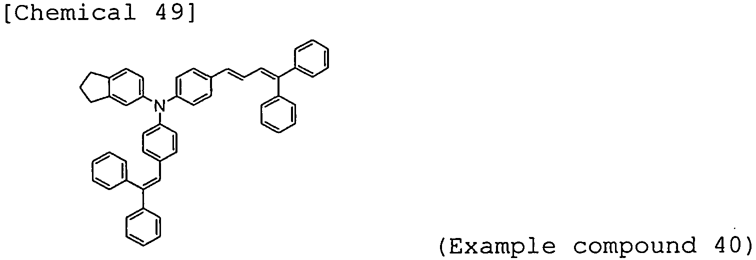

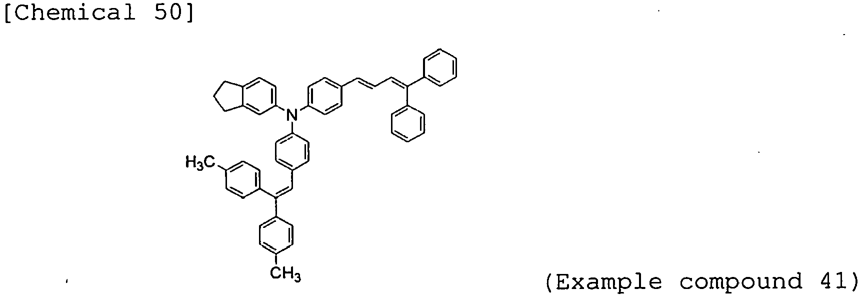

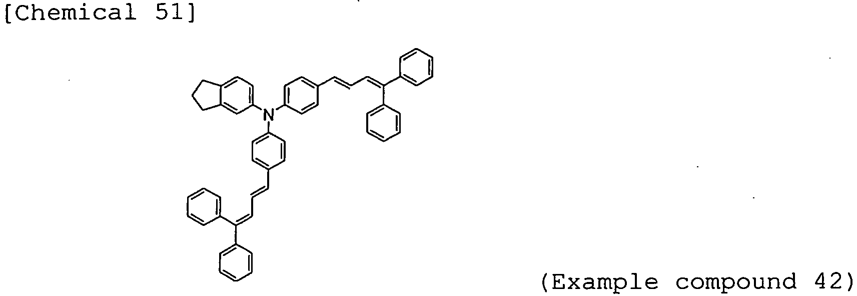

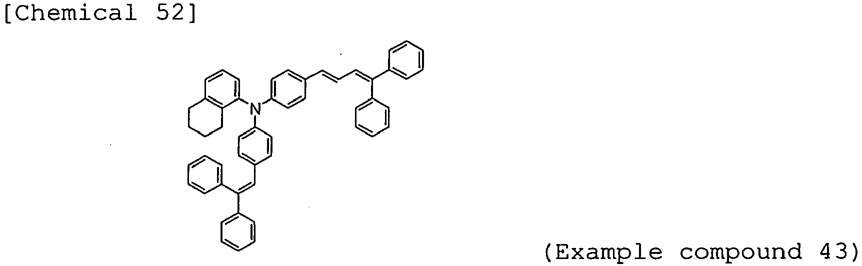

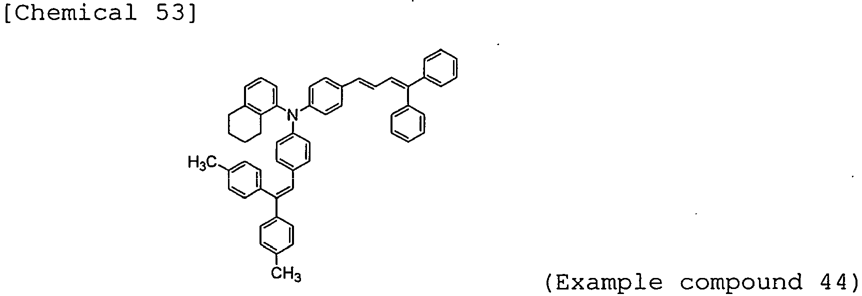

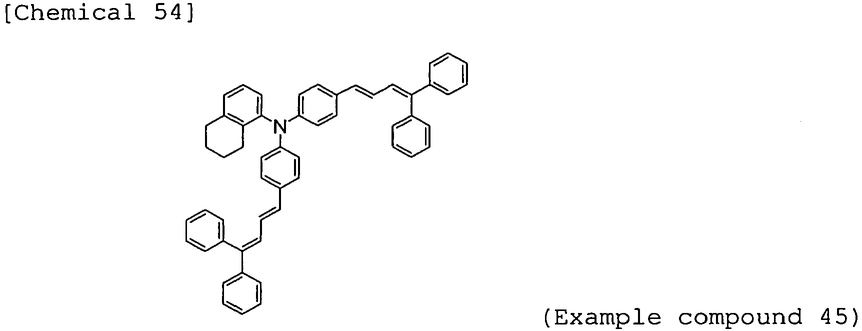

Among the triphenylamine derivatives represented by the general formula (1), the triphenylamine derivative represented by the above general formula (2) or the general formula (3) exhibits particularly excellent electric charge transporting property and is most desirably used as a charge transporting agent for the production of an organic photosensitive material for electrophotography.

### <Preparation of triphenylamine derivatives>

The triphenylamine derivatives of the invention mentioned above can be synthesized by using a triphenylamine compound represented by the following general formula (4) as a starting material. 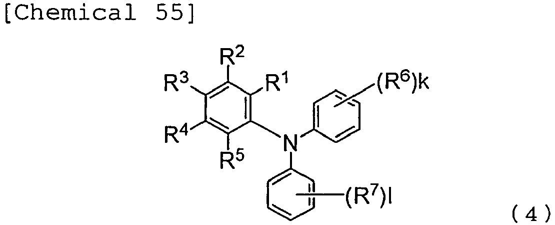 In the above general formula (4), R¹ to R⁷, k and 1 are
as defined in the general formula (1).

The above triphenylamine compound has been known as disclosed in, for example, JP-A-9-292723 (patent document 10). The triphenylamine derivative of the invention represented by the above general formula (1) is prepared by introducing the group X¹ into the triphenylamine compound and, next, by introducing the group X² therein.

### (Introduction of the group X¹)

To introduce the group X¹ into the triphenylamine compound of the general formula (4), first, a carbonyl group (formyl group or ketone group) is introduced to a benzene ring bonded to an N atom of the above compound to thereby synthesize a carbonyl compound represented by the following general formula (5) or (5').

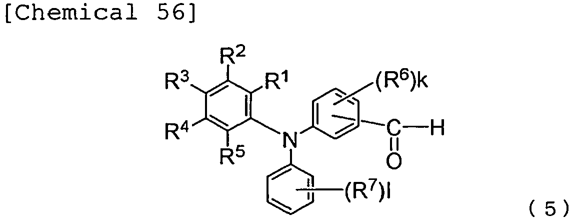

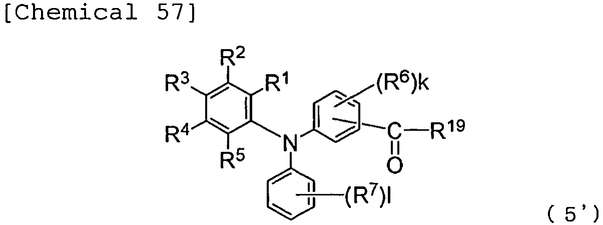

In the above general formulas (5) and (5'),
R¹ to R⁷, k and 1 are as defined in the general formula (1),
R¹⁹ is an alkyl group having 1 to 6 carbon atoms, an aromatic hydrocarbon group, a condensed polycyclic aromatic group or an aromatic heterocyclic ring group.
R¹⁹ is a group corresponding to R⁸ (but excluding hydrogen atom) in the general formula (1a) that represents the group X¹.

Next, by utilizing the Wittig reaction, the carbonyl group (formyl group or ketone group) that is introduced is converted into the group X¹ represented by the general formula (1a) i.e., the group X¹ is thus introduced.

To obtain the carbonyl compound of the above general formula (5) by introducing the carbonyl group (formyl group) into the triphenylamine compound of the general formula (4), the triphenylamine compound may be reacted with a formylating agent such as N,N-dimethylformamide or N-methylformanilide in the presence of phosphorus oxychloride.
The above reaction is, usually, carried out by using a solvent inert to the reaction, such as o-dichlorobenzene or benzene. However, the formylating agent may be used in a very excess amount so as to serve as the reaction solvent.

Further, to obtain the carbonyl compound of the above general formula (5') by introducing the carbonyl group (ketone group) into the triphenylamine compound, the triphenylamine compound may be reacted with an acid chloride (R¹⁹COCl) in the presence of a Lewis acid such as aluminum chloride, iron chloride or zinc chloride. The above reaction is, usually, carried out by using a solvent inert to the reaction, such as nitrobenzene, dichloromethane or carbon tetrachloride.

To convert the carbonyl group into the group X¹ in the carbonyl compound of the above general formula (5) or (5') by utilizing the Wittig reaction, the carbonyl compound may be treated with a triphenylphosfine and with a halogen compound represented by the following general formula (6), 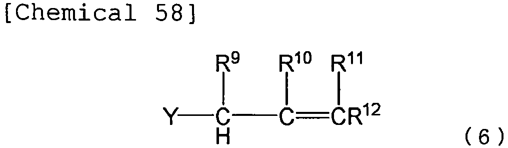 Wherein,
R⁹ to R¹² are as defined in the general formula (1a), and
Y is a halogen atom such as chlorine atom or bromine atom.
Due to the above reaction, the group X¹ is introduced into the triphenylamine compound of the above general formula (4). Namely, a compound represented by the following general formula (7) is obtained.

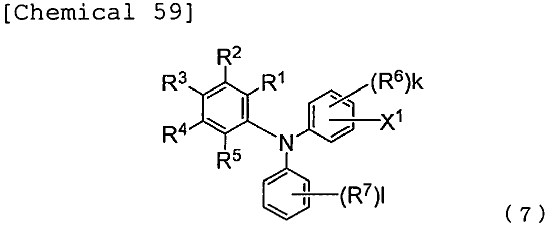

In the above general formula (7), R¹ to R⁷, k, l and X¹ are as defined in the general formula (1).

The above reaction (Wittig reaction) is carried out by using an organic solvent inert to the reaction, such as N,N-dimethylformamide, N,N-dimethylacetamide, tetrahydrofurane, dioxane, benzene or toluene.
Instead of using the above halogen compound and the triphenylphosfine, further, the carbonyl compound of the above general formula (5) or (5') may be reacted with a Wittig reagent obtained by acting a trialkoxyphosphorus compound on the halogen compound.

It is desired that the reaction temperature in the above Wittig reaction is in a range of from 10 to 200°C and, specifically, from 20 to 100°C.
It is, further, desired that the Wittig reaction is carried out in the presence of a basic catalyst such as n-butyllithium, phenyllithium, sodium methoxide, sodium ethoxide or potassium tert-butoxide.

### (Introduction of the group X²)

To introduce the group X² into the compound of the general formula (7) to which the group X¹ has been introduced in a manner as described above, the carbonyl group (formyl group or ketone group) is introduced therein in the same manner as that of introducing the group X¹ to form a carbonyl compound and, thereafter, the carbonyl group is converted into the group X² by the Wittig reaction.

Namely, the formyl group or the ketone group is introduced into the compound of the general formula (7) in the same manner as described above to synthesize a compound represented by the following general formula (8); 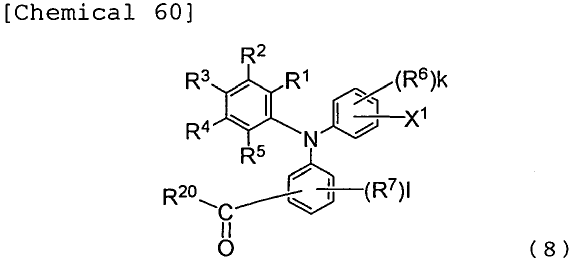 wherein,
R¹ to R⁷, k, 1 and X¹ are as defined in the general formula (1), and
R²⁰ is a hydrogen atom, an alkyl group having 1 to 6 carbon atoms, an aromatic hydrocarbon group, a condensed polycyclic aromatic group or an aromatic heterocyclic ring group.
If formylation is conducted during the reaction, R²⁰ becomes a hydrogen atom. If a ketone is obtained by using an acid chloride (R²⁰COCl), then R²⁰ becomes a group other than the hydrogen atom.
The above group R²⁰ is a group corresponding to R¹³ or R¹⁵ in the general formula (1b) that represents the group X².

The carbonyl compound of the general formula (8) obtained as described above is subjected to the Wittig reaction in the same manner as when introducing the group X¹ to thereby introduce the group X² and, therefore, to obtain the triphenylamine derivative of the present invention represented by the general formula (1) and, specifically, by the general formula (2) or (3).

The Wittig reaction uses a halogen compound represented by the following general formula (9) or (9');

Y-CH(R¹⁴) -C (R¹⁵) =CR¹⁶R¹⁷ (9)

Y-CH(R¹⁶) (R¹⁷) (9')

Wherein,
Y is a halogen atom such as chlorine atom or bromine atom, and
R¹⁴ to R¹⁷ are as defined in the above general formula (1b),
or uses a Wittig reagent derived from the above halogen compound instead of using the halogen compound of the above general formula (6).

In introducing the above group X¹ or the group X², the carbonyl group (formyl group) can also be introduced by introducing a halogen atom into the benzene ring relying on the halogenation reaction known per se. followed by the reaction with magnesium or lithium to obtain an organometal compound thereof and, thereafter, by reacting the organometal compound with an N,N-dimethylformamide.
The above halogenation reaction has been described in detail, for example, in the fourth series of Experimental Chemistry 19 (pp. 363-482, The Chemical Society of Japan, 1992). The reaction of the organometal compound with the dimethylformamide has been described in detail, for example, in the fourth series of Experimental Chemistry 21 (pp. 23-44 and pp. 179-196, The Chemical Society of Japan, 1991).

The compound having a double bond newly formed by the above Wittig reaction is obtained as a cis-form, a trans-form or a mixture of the cis-form and the trans-form. In the triphenylamine derivative of the invention, therefore, the double bonds of the group X¹ and the group X² in the general formula (1) represents any of the cis-form, trans-form, or a mixture of the cis-form and the trans-form.

After the reaction, the refining can be conducted by the adsorptive refining by using column chromatography, silica gel, active carbon or activated earth, or by the recrystallization or crystallization by using a solvent.
Further, the obtained compound can be identified by the IR measurement or by the elemental analysis.

The thus obtained triphenylamine derivative of the present invention has a high charge mobility and can be preferably used as a charge transporting agent in the organic photosensitive material for electrophotography. It can be, further, used as a material for organic electroluminescence (EL) devices.

### <Organic photosensitive material for electrophotography>

The organic photosensitive material that uses the triphenylamine derivative of the invention as a charge transporting agent is the one that comprises an electrically conducting substrate on which there is formed a photosensitive layer that contains the above charge transporting agent as well as the charge generating agent. Here, the organic photosensitive material can be realized in two types of either the one in which the photosensitive layer is a single layer containing the charge transporting agent and the charge generating agent (photosensitive layer of the single-layer type) or the one in which the photosensitive layer comprises a charge transporting layer containing the charge transporting agent and a charge generating layer containing the charge generating agent (photosensitive layer of the lamination type).

As the electrically conducting substrate for supporting the photosensitive layer, there can be used an electrically conducting material that has been used in the known photosensitive materials for electrophotography. Concretely, there can be used a sheet of a metal such as copper, aluminum, silver, iron, zinc or nickel or an alloy thereof, or a drum made of the above sheet. Or there can be used those obtained by vacuum-evaporating or electrolytically plating these metals onto a plastic film or a cylinder, or by applying or vacuum-evaporating a layer of an electrically conducting compound such as electrically conducting polymer, indium oxide or tin oxide onto a glass, a paper or a plastic film.

Depending upon the type of the photosensitive layer, the photosensitive layer can be formed on the electrically conducting substrate by the vacuum evaporation (in the case of the lamination type photosensitive layer). Usually, however, the photosensitive layer is formed on the electrically conducting substrate by using a resin binder. That is, the charge transporting agent and the charge generating agent are dissolved in an organic solvent together with the resin binder to prepare a coating solution which is then applied onto the electrically conducting substrate and is dried to form a photosensitive layer of the single-layer type or the lamination type.

As the resin binder used for forming the photosensitive layer, there can be used a thermoplastic or thermosetting resin that has heretofore been used for forming the photosensitive layers. Concrete examples thereof include meth(acrylic) resins such as polyacrylate and polymethacrylate, as well as polyamide resin, acrylonitrile resin, vinyl chloride resin, acetal resin, butylal resin, vinyl acetate resin, polystyrene resin, polyolefin resin, cellulose ester, phenol resin, epoxy resin, polyester, alkyd resin, silicone resin, polycarbonate resin, polyurethane resin and polyimide resin. In addition to them, there can be, further, used, as resin binders, organic photoelectrically conducting polymers such as polyvinyl carbazole, polyvinyl anthracene and polyvinyl pyrene.

The above resin binders are used in one kind or in a combination of two or more kinds. As the binder resin for the charge transporting layer in the lamination type photosensitive layer, in particular, the invention preferably uses a polycarbonate resin and, specifically, a polycarbonate resin having a recurring unit represented by the following formula (A), 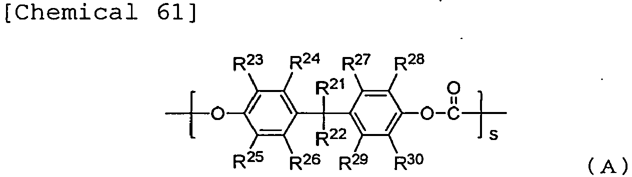 wherein,
R²¹ and R²² may be the same or different, and are hydrogen atoms, alkyl groups having 1 to 4 carbon atoms, alkoxy groups having 1 to 4 carbon atoms, or phenyl groups for which a halogen atom may be substituted, and may form a ring together,
R²³ to R³⁰ may be the same or different, and are hydrogen atoms, halogen atoms, alkyl groups having 1 to 6 carbon atoms, alkoxy groups having 1 to 6 carbon atoms or phenyl groups, and
s is a positive integer.

Among the polycarbonate resins having the recurring unit represented by the above formula (A), preferred polycarbonate resins are as exemplified below.

(1) Polycarbonate resins of the type of bisphenol A (e.g., Eupilon E-Series manufactured by Mitsubishi Gas Chemical Company, Inc.) having a recurring unit represented by the following formula (B); 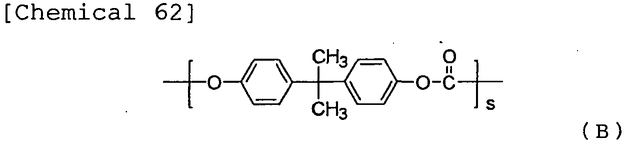 Wherein,
   s is a positive integer.

(2) Polycarbonate resins of the type of bisphenol Z (e.g., Eupilon Z-series manufactured by Mitsubishi Gas Chemical Company, Inc.) having a recurring unit represented by the following formula (C); 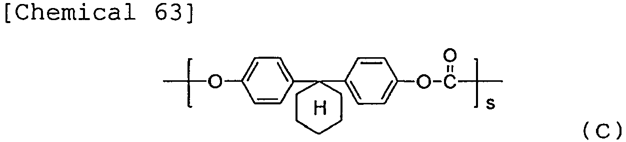
Wherein,
s is a positive integer.

(3) Copolymerized polycarbonate resins containing bisphenol A, bisphenol Z and biphenol as structural units.
   Such copolymerized polycarbonate resins have been disclosed in, for example, JP-A-4-179961. There can be exemplified, for example, polycarbonate resins of the bisphenol/biphenol type represented by the following formula (D) ; 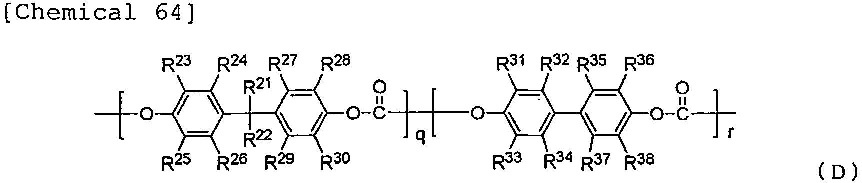
wherein,
R²¹ to R³⁰ are the same as R²¹ to R³⁰ in the above-mentioned formula (A),
R³¹ to R³⁸ may be the same or different, and are hydrogen atoms, halogen atoms, alkyl groups having 1 to 6 carbon atoms, alkoxy groups having 1 to 6 carbon atoms or phenyl groups,
R³¹ and R³², R³³ and R³⁴, R³⁵ and R³⁶, and R³⁷ and R³⁸ together may form rings, respectively, and
q and r are mole numbers of the above recurring units and, preferably, numbers satisfying q/(q + r) = 0.1 to 0.9.

Among the above copolymerized polycarbonate resins, particularly preferred example is a polycarbonate resin of the bisphenol A/biphenol type represented by the following formula (E) ; 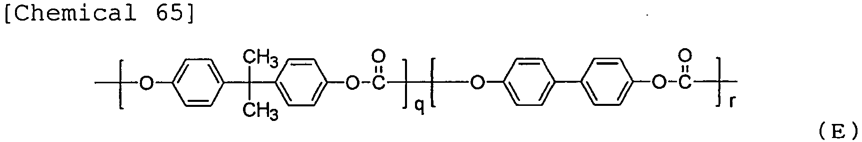 wherein,
q and r are mole numbers of the above recurring units, and q/(q + r) = 0.85.

In addition to the polycarbonate resins having a recurring unit of the above formula (A), there can be preferably used polycarbonate resins having recurring units of the following formulas (F) to (I).

(4) Polycarbonate resins having a recurring unit represented by the following formula (F); 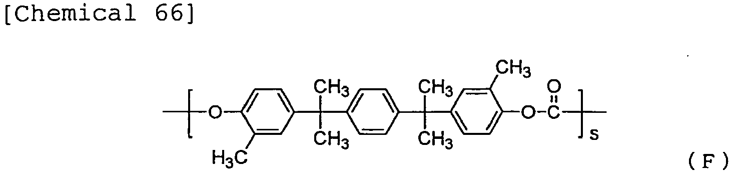 Wherein,
   s is a positive integer.
   The above copolymerized polycarbonate resins have been disclosed in, for example, JP-A-6-214412.

(5) Polycarbonate resins having a recurring unit represented by the following formula (G); 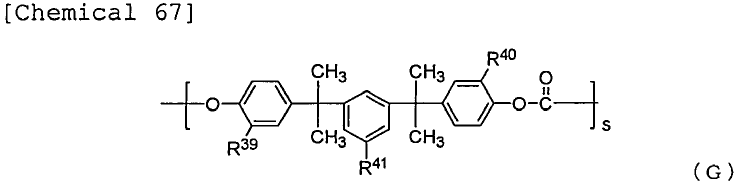 wherein,
   R³⁹' R⁴⁰ and R⁴¹ may be the same or different, and are hydrogen atoms, halogen atoms, alkyl groups having 1 to 6 carbon atoms, cycloalkyl groups, aromatic hydrocarbon groups, condensed polycyclic aromatic groups or alkyl groups substituted with an aromatic hydrocarbon group or a condensed polycyclic aromatic group, and
   s is a positive integer.
   The above copolymerized polycarbonate resins have been disclosed in, for example, JP-A-6-222581.

(6) Polycarbonate resins of the siloxane type having a recurring unit represented by the following formula (H); 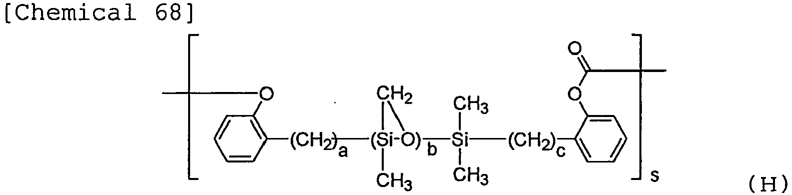 or by the following formula (I); 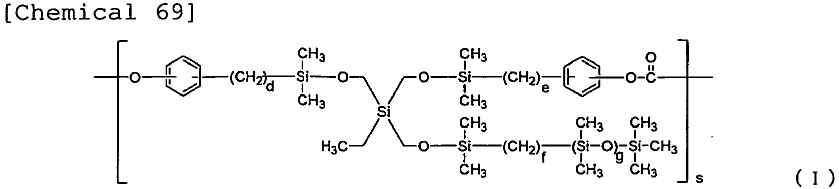
Wherein,
a to g and s are positive integers.
The above copolymerized polycarbonate resins have been disclosed in, for example, JP-A-5-088398 (patent document 3) and JP-A-11-065136.

As the organic solvent used for the preparation of a coating solution for forming the photosensitive layer, there can be used any organic solvent without special limitation provided it is capable of dissolving the charge transporting agent (e.g., triphenylamine derivatives of the general formula (1)) and the above-mentioned resin binder added thereto and is capable of, further, dissolving or dispersing the charge generating agent. Usually, there are used those solvents exemplified below in one kind or in a combination of two or more kinds.

Alcohols such as methanol, ethanol and 2-propanol;
ketones such as acetone, methyl ethyl ketone and cyclohexanone;
amides such as N,N-dimethylformamide and N,N-dimethylacetamide;
sulfoxides such as dimethyl sulfoxide;
ethers such as tetrahydrofurane, dioxane, dioxorane, ethylene glycol dimethyl ether, diethyl ether, diisopropyl ether, tert-butylmethyl ether;
esters such as ethyl acetate and methyl acetate;
aliphatic halogenated hydrocarbons such as methylene chloride, chloroform, 1,2-dichloroethane, dichloroethylene, carbon tetrachloride and trichloroethylene;
aromatic halogenated hydrocarbons such as chlorobenzene and dichlorobenzene;
aromatic hydrocarbons such as benzene, toluene and xylene; and
aliphatic hydrocarbons such as pentane, hexane, heptane, octane and cyclohexane.

Depending upon the form of the photosensitive layer that is to be formed, the coating solution is prepared by using the above organic solvent and dissolving or dispersing the charge generating agent and the charge transporting agent as well as a resin binder in the organic solvent.
That is, when the photosensitive layer of the single-layer type is to be formed, the coating solution is prepared by adding the charge transporting agent, charge generating agent and resin binder to the organic solvent.
Further, when the photosensitive layer of the lamination type is to be formed, there are prepared a coating solution for the charge transporting layer by adding the charge transporting agent and resin binder to the organic solvent, and a coating solution for the charge generating layer by adding the charge generating agent and resin binder to the organic solvent.

As required, further, the above various solutions may contain various additives in an attempt to further improve stability and applicability of the coating solution and to improve charge properties and durability of the photosensitive layer.
As the additives, there can be exemplified plasticizers such as biphenylene-type compound, m-phenyl compound and dibutyl phthalate; surface lubricating agents such as silicone oil, grafted silicone polymer and various fluorocarbons; potential stabilizers such as dicyanovinyl compound and carbazole derivatives; monophenol-type antioxidants such as 2,6-di-tert-butyl-4-methylphenol and the like; bisphenol-type antioxidants; amine-type antioxidants such as 4-diazabicyclo[2,2,2]octane and the like; salicylic acid-type antioxidants; antioxidants such as tocophenol and the like; ultraviolet ray absorber; sensitizing agent; and the like.
These additives can be suitably used in amounts in ranges in which they do not impair the properties of the photosensitive layer or the applicability of the coating solution.

The above coating solution can be applied by a known coating method such as dip-coating method, spray-coating method, spinner-coating method, Meyer's bar-coating method, blade-coating method, roller-coating method or curtain-coating method.

A desired photosensitive layer is formed by drying a coating layer formed by applying the above coating solution. Here, in the case of the lamination type photosensitive layer, the charge generating layer or the charge transporting layer is formed on the electrically conducting substrate and, thereafter, the charge transporting layer or the charge generating layer is formed thereon.
The above drying is desirably conducted by maintaining the coating layer at room temperature followed by heating. The heating is desirably conducted at a temperature of 30 to 200°C for a period of time in a range of 5 minutes to 2 hours without blowing the air or by blowing the air.

Prior to forming the photosensitive layer, it is also allowable to form an underlying layer on the electrically conducting substrate and form the photosensitive layer on the underlying layer. The underlying layer is for improving the barrier function for preventing deterioration of the surface of the electrically conducting substrate and for improving close adhesion between the photosensitive layer and the surface of the electrically conducting substrate. The underlying layer is formed of a thin resin layer such as of polyvinyl alcohol, nitrocellulose, casein, ethylene/acrylic acid copolymer, polyamide, e.g., nylon, polyurethane or gelatin, or is formed of an aluminum layer or a resin layer in which a metal oxide such as titanium oxide or the like is dispersed.
The thickness of the underlying layer is, desirably, in a range of from 0.1 to 5 *µ*m and, specifically, from 0.5 to 3 *µ*m. This is because if the underlying layer has a too large thickness, inconvenience occurs such as an increase in the residual potential in the photosensitive material due to an increase in the resistivity.

On the thus formed photosensitive layer, there can be further suitably formed a protection layer for preventing the photosensitive layer from being deteriorated by ozone or nitrogen oxide or from being worn out.

In the present invention, as described above, a triphenylamine derivative of the general formula (1) is used as the charge transporting agent in the photosensitive layer that is formed as described above. The amount of use of the triphenylamine derivative may differ depending upon the kind of the photosensitive layer that is formed, but is, usually, in a range of 10 to 1000 parts by weight, preferably, 30 to 500 parts by weight and, more preferably, 40 to 200 parts by weight per 100 parts by weight of the resin binder. The triphenylamine derivative is, desirably, present in the single-layer type photosensitive layer or in the charge transporting layer of the lamination type photosensitive layer.

As required, the photosensitive layer may, further, use other charge transporting agents than the above triphenylamine derivative in amounts in a range in which they will not impair excellent properties of the triphenylamine derivative.

The above other charge transporting agents have been known as represented, for example, by the following compounds.

Other charge transporting agents:
(1)A hydrazone compound represented by the following general formula (10); 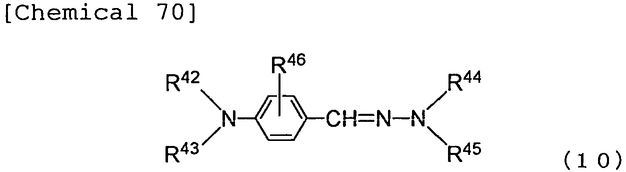
wherein,
R⁴² and R⁴³ may be the same or different, and are lower alkyl groups having 1 to 4 carbon atoms, aromatic hydrocarbon groups, condensed polycyclic aromatic groups or aralkyl groups,
R⁴⁴ and R⁴⁵ may be the same or different, and are lower alkyl groups having 1 to 4 carbon atoms, aromatic hydrocarbon groups, condensed polycyclic aromatic groups, aralkyl groups or heterocyclic groups, and R⁴⁴ and R⁴⁵ together may form a ring,
R⁴⁶ is a hydrogen atom, a lower alkyl group having 1 to 4 carbon atoms, an aromatic hydrocarbon group, a condensed polycyclic aromatic group, an aralkyl group, a lower alkoxy group having 1 to 4 carbon atoms or a halogen atom, and R⁴⁶ and R⁴² or R⁴³ together may form a ring.
The above hydrazone compound has been disclosed in, for example, JP-B-55-042380 (patent document 6), JP-A-60-340999 (patent document 11) and JP-A-61-023154 (patent document 12).

(2) A triphenylamine dimer represented by the following general formula (11); 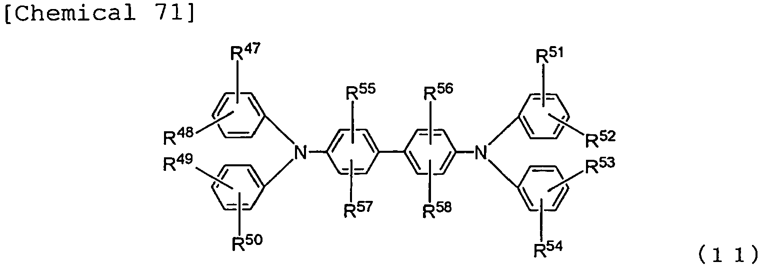 wherein,
   R⁴⁷ to R⁵⁸ may be the same or different, and are hydrogen atoms, lower alkyl groups having 1 to 4 carbon atoms, lower alkoxy groups having 1 to 4 carbon atoms, halogenoalkyl groups having 1 to 4 carbon atoms, halogenoalkoxy groups having 1 to 4 carbon atoms, aromatic hydrocarbon groups, condensed polycyclic aromatic groups or halogen atoms.
   The above triphenylamine dimer has been disclosed in, for example, JP-B-58-032372 (patent document 13).

(3) A distyryl compound represented by the following general formula (12); 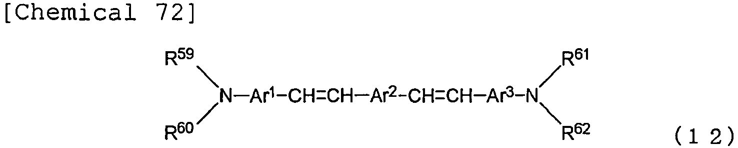 wherein,
   R⁵⁹ to R⁶² may be the same or different, and are lower alkyl groups having 1 to 4 carbon atoms, aromatic hydrocarbon groups or condensed polycyclic aromatic groups,
   Ar¹ and Ar³ *may be the same or different, and are phenylene groups, and Ar² is a divalent group of a monocyclic or polycyclic aromatic hydrocarbon having 4 to 14 carbon atoms or a divalent group of an aromatic heterocyclic ring, and
   a substituent which Ar¹, Ar² and Ar³ may possess is a group selected from a lower alkyl group having 1 to 4 carbon atoms, a lower alkoxy group having 1 to 4 carbon
   atoms, an aryloxy group and a halogen atom.
   The above distyryl compound has been disclosed in, for example, U.S. Patent No. 3873312 (patent document 14).

(4) Charge transporting agents other than those described above;
Tetraphenylbutadiene compounds,
α-phenylstylbene compounds,
polyvinylcarbazole compounds,
triphenylmethane compounds.

The charge generating agents to be added to the photosensitive layer are the materials that absorb light and generate electric charge highly efficiently, and can be roughly grouped into the inorganic charge generating agents and the organic charge generating agents.
As the inorganic charge generating agents, there have been known selenium, selenium-tellurium and amorphous silicon.
As the organic charge generating agents, there have been known cationic dyes (e.g., thiapyrylium salt dye, azulenium salt dye, thiacyanine dye, quinocyanine dye), squalium salt pigment, phthalocyanine pigment, polycyclic quinone pigments (e.g., anthanthrone pigment, dibenzpylenequinone pigment, pyranthrone pigment), indigo pigment, quinacridone pigment, azo pigment, pyrrolopyrrole pigment and perylene pigment.
In the present invention, any of the above inorganic charge generating agents and organic charge generating agents can be respectively used in one kind or in a combination of two or more kinds. Among them, the organic charge generating agent is specifically preferred.

Among the organic charge generating agents, specifically preferred examples are phthalocyanine pigment, azo pigment, perylene pigment and polycyclic quinone pigment. Described below are concrete examples thereof.

Concrete examples of the phthalocyanine pigment include alkoxytitanium phthalocyanine (Ti(OR)₂Pc), oxotitanium phthalocyanine (TiOPc), copper phthalocyanine (CuPc), metal-free phthalocyanine (H₂Pc), hydroxygallium phthalocyanine (HOGaPc), vanadyl phthalocyanine (VOPc) and chloroindium phthalocyanine (ClInPc). More specifically, as the TiOPc, there can be exemplified α-type TiOPc, *β*-type TiOPc, y -type TiOPc, m-type TiOPc, Y-type TiOPc, A-type TiOPc, B-type TiOPc and TiOPc amorphous. As the H₂Pc, there can be exemplified α-type H₂Pc, *β*-type H₂Pc, τ -type H₂Pc and x-type H₂Pc .

As the azo pigment, there can be exemplified monoazo compounds, bisazo compounds and trisazo compounds. Specifically preferred are the bisazo compounds represented by the following structural formulas (J) to (L) and the trisazo compound represented by the following structural formula (M).

Bisazo compound of the structural formula (J); 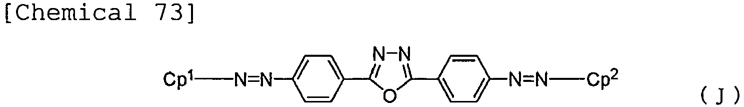 wherein Cp¹ and Cp² may be the same or different, and are the groups represented by the following formula (13) or (14).

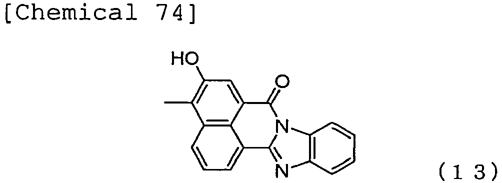

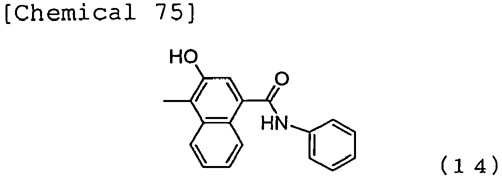

Bisazo compound of the structural formula (K);

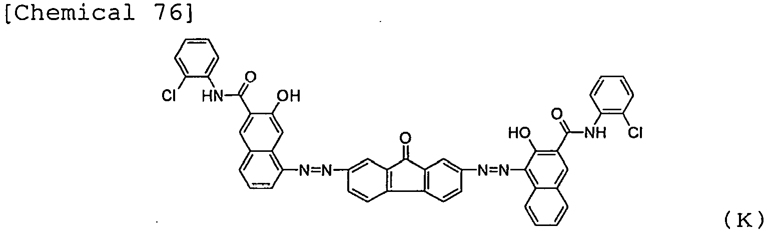

Bisazo compound of the structural formula (L); 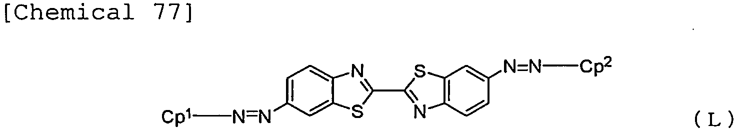 wherein Cp¹ and Cp² may be the same or different, and are the groups represented by the above formula (13) or (14).

Trisazo compound of the structural formula (M); 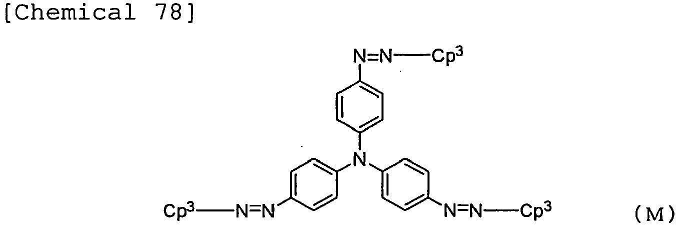 wherein Cp³ is a group represented by the following structural formula (15).

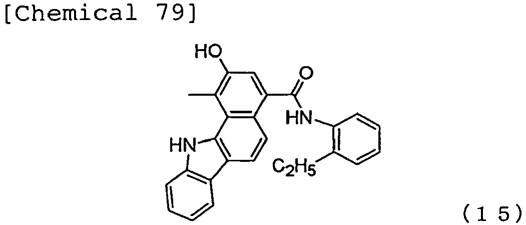

As the perylene compound and the polycyclic quinone pigment, further, the compounds represented by the following structural formulas (N) and (O) are specifically preferred. Structural formula (N); 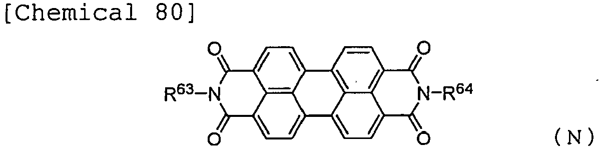 wherein R⁶³ and R⁶⁴ may be the same or different, and are lower alkyl having 1 to 4 carbon atoms, aromatic hydrocarbon groups or condensed polycyclic aromatic groups.
Structural formula (O);

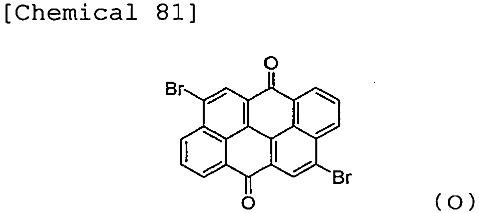

The ratio of the charge generating agent occupying the photosensitive layer may differ depending upon the type of the photosensitive layer, but is, usually, from 0.2 to 40 parts by mass and, specifically, from 0.5 to 20 parts by mass per 100 parts by mass of the resin binder in the case of the single-layer type photosensitive layer, and is from 30 to 400 parts by mass and, specifically, from 60 to 300 parts by mass per 100 parts by mass of the resin binder in the case of the charge generating layer of the lamination type photosensitive layer.

In the case of the single-layer type photosensitive layer, the thickness of the photosensitive layer is from about 5 to about 100 µm and, specifically, from about 15 to about 45 µ m.
In the case of the lamination type photosensitive layer, the thickness of the charge generating layer is from about 0.01 to about 5 µm and, specifically, from about 0.05 to about 2 µm while the thickness of the charge transporting layer is from about 5 to about 40 µm and, specifically, from about 10 to about 30 µm.

Upon coming into electric contact with the charge generating layer, the charge transporting layer in the lamination type photosensitive layer receives charge carriers that are injected from the charge generating layer in the presence of an electric field and transports the charge carriers to the surface of the photosensitive layer. Here, the charge transporting layer may be laminated on the charge generating layer or may be laminated thereunder. From the standpoint of suppressing the deterioration of the charge generating layer, however, it is desired that the charge transporting layer is laminated on the charge generating layer.

The organic photosensitive material for electrophotography includes the photosensitive layer that contains the triphenylamine derivative of the above general formula (1) as the charge transporting agent, and effectively avoids the precipitation of crystals and occurrence of pinholes during the formation of the photosensitive layer owing to excellent properties of the triphenylamine derivative. Besides, the above organic photosensitive material is highly sensitive, has a low residual potential, and is capable of forming vivid images over extended periods of time even when the images are repetitively formed by the electrophotography.

To form the image by using the organic photosensitive material by the electrophotography, the surface of the photosensitive material is electrically charged to a predetermined polarity by using, for example, a corona charger and is, next, irradiated with light (exposure to image-bearing light) based on the image data to form an electrostatic latent image, and the electrostatic latent image thus formed is developed by using a developing agent known per se. to form a toner image on the surface of the photosensitive material. The toner image is then transferred onto a predetermined recording material, and the transferred toner image is fixed onto the recording material by the application of heat and pressure. Afer the toner image has been transferred, the surface of the photosensitive material is irradiated with light to remove the electric charge. Further, the toner remaining thereon is removed by using a cleaning blade or the like and is used for the image-forming processing of the next time.

### EXAMPLES

The invention will now be concretely described by way of Examples to which only, however, the invention is in no way limited.

### [Synthesis Example 1 (synthesis of Example compound No. 1)]

A compound represented by the following structural formula (16) (see patent document 10) was provided as a starting material.

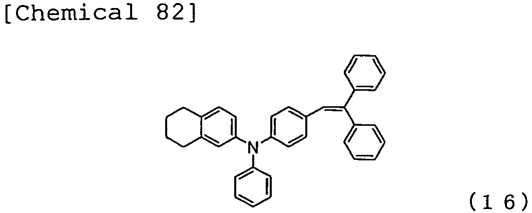

15 Grams of the above compound, 4.5 g of N,N-dimethylformamide and 8 g of toluene were added into a reaction vessel, and to which was, further, added 9 g of a phosphoryl trichloride dropwise. The mixture was stirred being heated at 80°C for 3 hours, left to cool, and to which 8 g of water was added dropwise while being cooled followed by the addition of sodium carbonate to render the reaction solution to be alkaline.
Next, after heated at 60°C for 3 hours, the reaction product was extracted with toluene. After washed with water and, then, with saturated brine, the solution was dried by using the magnesium sulfate. After that, the solvent was distilled off to obtain 14.4 g of a yellow solid formyl compound represented by the following structural formula (17).

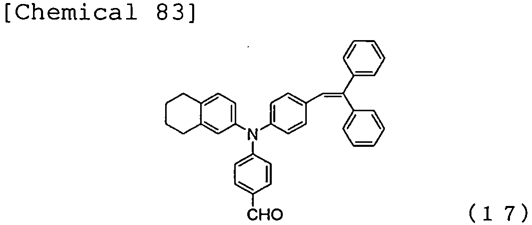

4 Grams of the obtained formyl compound and 10.4 g of a diphenylpropylenediethyl phosphorus ester were dissolved in 50 ml of the N,N-dimethylformamide, and to which 1.8 g of sodium methylate was added maintaining the temperature at 20 ± 5°C. After stirred for 2 hours, 30 ml of ion-exchanged water was added thereto, and the refining treatment was conducted in a customary manner to obtain 3.3 g of a yellow solid material (yield, 61%).

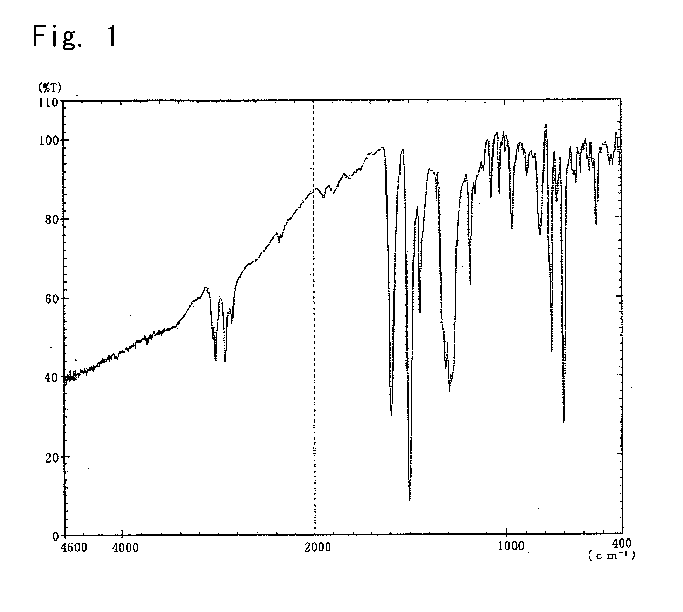

The obtained yellow solid material was identified for its structure by the elemental analysis and IR measurement. The IR spectrum thereof was as shown in Fig. 1.
Further, the elementally analyzed values were as follows:

| | Carbon | Hydrogen | Nitrogen |
|---|---|---|---|
| Measured (%) | 91.71% | 6.41% | 1.88% |
| Calculated (%) | 91.59% | 6.36% | 2.05% |

The above results tell that the obtained yellow solid material corresponds to the above-mentioned Example compound No. 1, and is a compound represented by the following formula.

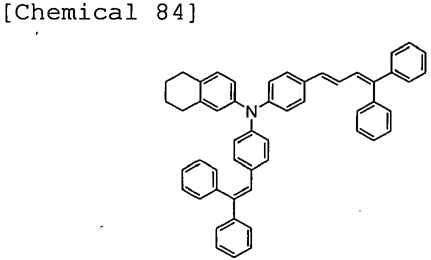

### [Synthesis Example 2 (synthesis of Example compound No. 2)]

A compound represented by the following structural formula (18) (see patent document 15) was provided as a starting material.

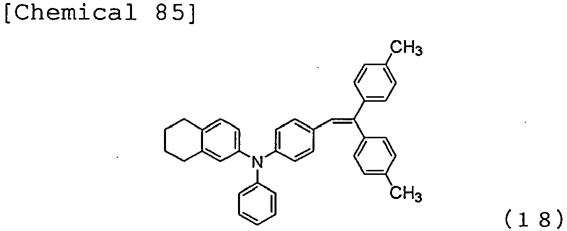

16 Grams of the above compound, 4.5 g of N,N-dimethylformamide and 8 g of toluene were added into the reaction vessel, and to which was, further, added 9 g of a phosphoryl trichloride dropwise. The mixture was stirred being heated at 80°C for 3 hours, left to cool, and to which 8 g of water was added dropwise while being cooled followed by the addition of sodium carbonate to render the reaction solution to be alkaline.
Next, after heated at 60°C for 3 hours, the reaction product was extracted with toluene. After washed with water and, then, with saturated brine, the solution was dried by using the magnesium sulfate. After that, the solvent was distilled off to obtain 14.1 g of a yellow solid formyl compound represented by the following structural formula (19).

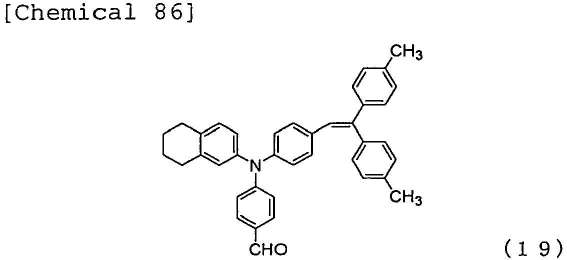

4 Grams of the obtained formyl compound and 10.4 g of the diphenylpropylenediethyl phosphorus ester were dissolved in 50 ml of the N,N-dimethylformamide, and to which 1.7 g of sodium methylate was added while maintaining the temperature at 20 ± 5°C. After stirred for 2 hours, 30 ml of ion-exchanged water was added thereto, and the refining treatment was conducted in a customary manner to obtain 2.9 g of a yellow solid material (yield, 54%).

The obtained yellow solid material was identified for its structure by the elemental analysis and IR measurement.
The elementally analyzed values were as follows:

| | Carbon | Hydrogen | Nitrogen |
|---|---|---|---|
| Measured (%) | 91.46% | 6.71% | 1.83% |
| Calculated (%) | 91.35% | 6.67% | 1.97% |

The above results tell that the obtained yellow solid material corresponds to the above-mentioned Example compound No. 2, and is a compound represented by the following formula.

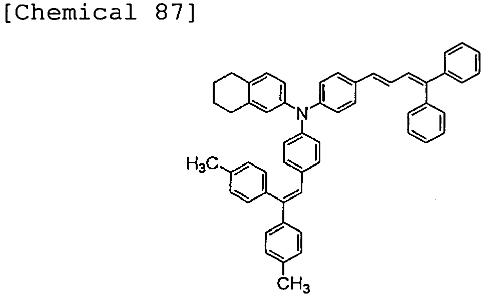

### [Synthesis Example 3 (synthesis of Example compound No. 10)]

A compound represented by the following structural formula (20) was provided as a starting material.

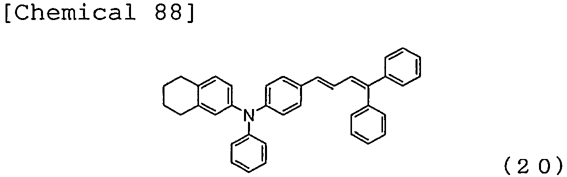

17 Grams of the above compound, 5.0 g of N,N-dimethylformamide and 9 g of toluene were added into the reaction vessel, and to which was, further, added 10 g of phosphoryl trichloride dropwise. The mixture was stirred being heated at 80°C for 3 hours, left to cool, and to which 9 g of water was added dropwise while being cooled followed by the addition of sodium carbonate to render the reaction solution to be alkaline.
Next, after heated at 60°C for 3 hours, the reaction product was extracted with toluene. After washed with water and, then, with saturated brine, the solution was dried by using the magnesium sulfate. After that, the solvent was distilled off to obtain 14.1 g of a yellow solid formyl compound represented by the following structural formula (21).

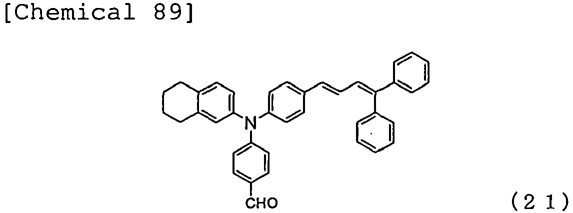

5 Grams of the obtained formyl compound and 13.5 g of the diphenylpropylenediethyl phosphorus ester were dissolved in 55 ml of the N,N-dimethylformamide, and to which 2.0 g of sodium methylate was added while maintaining the temperature at 20 ± 5°C. After stirred for 2 hours, 35 ml of ion-exchanged water was added thereto, and the refining treatment was conducted in a customary manner to obtain 3.2 g of a yellow solid material (yield, 48%).

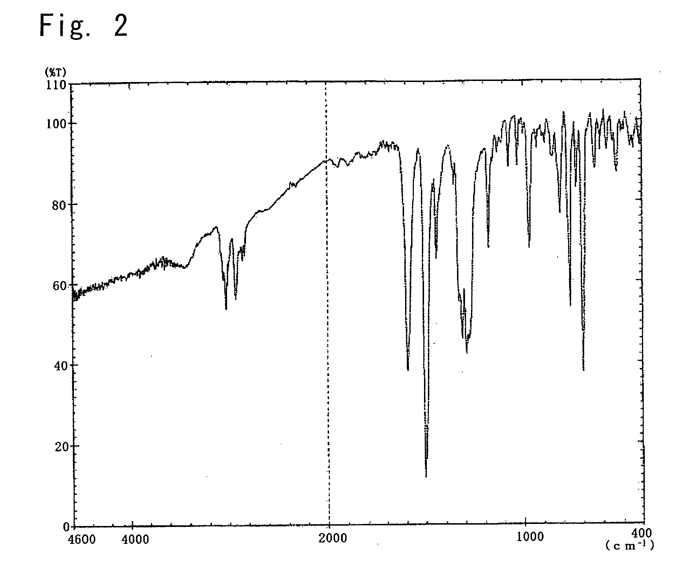

The obtained yellow solid material was identified for its structure by the elemental analysis and IR measurement. The IR spectrum thereof was as shown in Fig. 2.
Further, the elementally analyzed values were as follows:

| | Carbon | Hydrogen | Nitrogen |
|---|---|---|---|
| Measured (%) | 91.67% | 6.48% | 1.85% |
| Calculated (%) | 91.61% | 6.41% | 1.98% |

The above results tell that the obtained yellow solid material corresponds to the above-mentioned Example compound No. 10, and is a compound represented by the following formula,

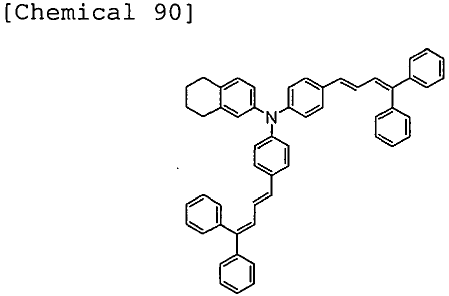

### [Photosensitive Material Example 1]

One part by mass of an alcohol-soluble polyamide (Amilan CM-4000, manufactured by Toray Industries, Inc.) was dissolved in 13 parts by mass of a methanol, and to which 5 parts by mass of a titanium oxide (Tipaque CR-EL, manufactured by Ishihara Sangyo Kaisha, Ltd.) was added. By using a paint shaker, the mixture thereof was dispersed for 8 hours to prepare a coating solution for undercoating layer. By using a wire bar, the coating solution was applied onto the aluminum surface of an aluminum-deposited PET film, and was dried under normal pressure at 60°C for one hour to form an undercoating layer having a thickness of 1 µm.

As a charge generating material, there was provided the following titanyl phthalocyanine (charge generating agent No. 1) having strong peaks at diffraction angles 2 θ ±0.2° of 9.6, 24.1 and 27.2 in the X-ray diffraction spectrum of Cu-Kα.

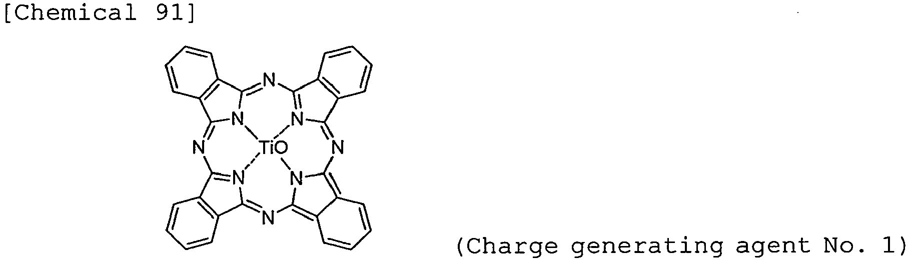

A polyvinyl butyral resin (S-LEC BL-S, produced by Sekisui Chemical Co, Ltd.) was provided as a binder resin for the charge generating layer.
1.5 Parts by mass of the above charge generating agent was added to 50 parts by mass of a cyclohexanone solution containing 3% of the polyvinyl butyral resin and was dispersed therein for one hour by using an ultrasonic dispersing machine. By using the wire bar, the obtained dispersion solution was applied onto the undercoating layer and was dried under normal pressure at 110°C for one hour to form a charge generating layer having a thickness of 0.6 µm.

Further, a polycarbonate resin (Eupilon Z, produced by Mitsubishi Engineering Plastic Co.) was provided as a binder resin for the charge transporting layer.
1.5 Parts by mass of the triphenylamine derivative (Example compound No. 1) synthesized in Example 1 was added as the charge transporting agent to 18.75 parts by mass of a dichloroethane solution containing 8.0% of the polycarbonate resin, and was completely dissolved therein by applying ultrasonic waves thereto.
By using the wire bar, the solution was applied onto the charge generating layer and was dried under normal pressure at 110°C for 30 minutes to form a charge transporting layer having a thickness of 20 µm to thereby prepare a photosensitive material No. 1.

### [Photosensitive Material Examples 2 and 3]

Photosensitive materials Nos. 2 and 3 were prepared in the same manner as in the Photosensitive Material Example 1 but using triphenylamine derivatives (Example compounds Nos. 2 and 10) synthesized in Examples 2 and 3 instead of using the charge transporting agent used in the Photosensitive Material Example 1.

### [Photosensitive Material Comparative Example 1]

For comparison, a photosensitive material No. 4 was prepared in the same manner as in the Photosensitive Material Example 1 but using a mixture of a comparative compound No. 1 and a comparative compound No. 2 represented by the following structures (mixing ratio: 2/1) instead of using the charge transporting agent used in the Photosensitive Material Example 1.

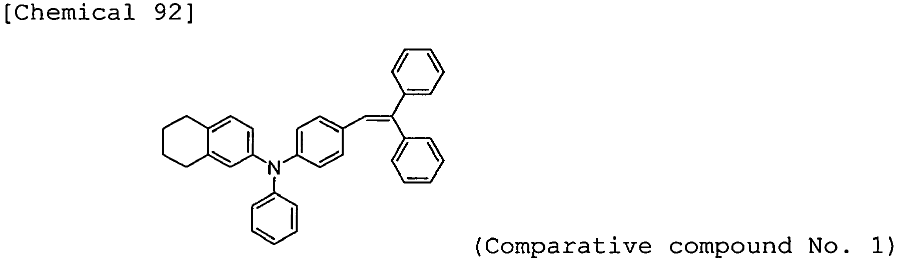

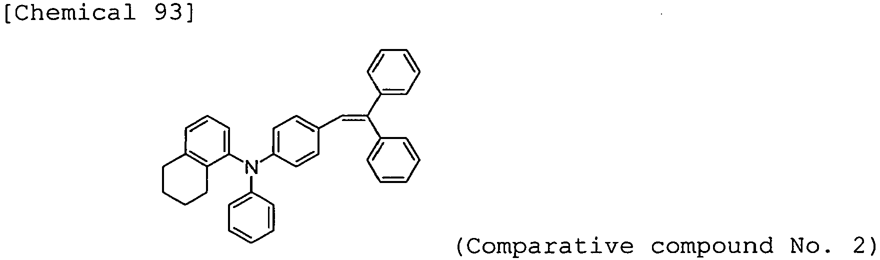

### (Evaluation of the electrophotographic properties of the photosensitive materials)

Photosensitive materials prepared in the Photosensitive Material Examples 1 to 3 and in the Photosensitive Material Comparative Example 1 were evaluated for their electrophotographic properties by using an electrostatic copying paper testing apparatus (trade name "EPA-8100A").
First, the photosensitive material was corona-charged at -5.5 kV in a dark place, and was measured for its charge potential V₀ at this moment.
Next, the photosensitive material was exposed to monochromatic light of 1.0 µW/cm² and 780 nm to find a half-value exposure E1/2 (µJ/cm²) and a residual potential Vr (-V) 2 seconds after the exposure to light. The results were as shown in Table 1.

**Table 1**

| Ex. and Comp. Ex. | Photosensitive material No. | Charged potential V₀ (-V) | Half-value exposure E₁/₂ (µJ/cm²) | Residual potential Vr (-V) |
|---|---|---|---|---|
| Ex. 1 | 1 | 753 | 0.25 | 34 |
| Ex. 2 | 2 | 750 | 0.25 | 32 |
| Ex. 3 | 3 | 749 | 0.24 | 26 |
| Comp. Ex. 1 | 4 | 760 | 0.27 | 51 |

The above results tell that the photosensitive materials for electrophotography having a photosensitive layer containing the triphenylamine derivatives of the invention as the charge transporting agent, feature low residual potentials.

### [Photosensitive Material Example 4]

As the charge generating material, there was provided a titanyl phthalocyanine (charge generating agent No. 2) having strong peaks at diffraction angles 2 θ ±0.2° of 7.5, 10.3, 12. 6, 22.5, 24.3, 25.4 and 28. 6 in the X-ray diffraction spectrum of Cu-Kα.

A charge generating layer having a thickness of 0.2 µ m was formed on the aluminum surface of an aluminum-deposited PET film in quite the same manner as in the Photosensitive Material Example 1 but using the above-mentioned charge generating agent.

Further, 0.9 parts by mass of the triphenylamine derivative (Example compound No. 25) of Example 1 was added to 7.38 parts by mass of a tetrahydrofurane solution containing 12.2% of the above-mentioned polycarbonate resin, and was completely dissolved therein by applying ultrasonic waves thereto.
By using the wire bar, the solution was applied onto the charge generating layer and was dried under normal pressure at 110°C for 30 minutes to form a charge transporting layer having a thickness of 10 µm. Thereafter, a semi-transparent metal electrode was vapor-deposited on the charge transporting layer to thereby prepare a photosensitive material No. 5.

### [Photosensitive Material Examples 5 and 6]

Photosensitive materials Nos. 6 and 7 were prepared in the same manner as in the Photosensitive Material Example 4 but using triphenylamine derivatives (Example compounds Nos. 2 and 10) of Examples 2 and 3 instead of using the charge transporting agent used in the Photosensitive Material Example 4.

### [Photosensitive Material Comparative Example 2]

For comparison, a photosensitive material No. 8 was prepared in the same manner as in the Photosensitive Material Example 4 but using the charge transporting agent (mixture of the Comparative compounds Nos. 1 and 2) used in the Photosensitive Material Comparative Example 1 instead of using the charge transporting agent used in the Photosensitive Material Example 4.

### [Drift mobility]

The photosensitive materials prepared in the Photosensitive Material Examples 4 to 6 and in the Photosensitive Material Comparative Example 2 were measured for their drift mobilities. The measurement was taken based on the time-of-flight method at 2 x 10⁵ V/cm. The results were as shown in Table 2.

**Table 2**

| Ex. and Comp. Ex. | Photosensitive material No. | Drift mobility (cm²/V•s) |
|---|---|---|
| Ex. 4 | 5 | 4.4 × 10⁻⁵ |
| Ex. 5 | 6 | 4.6 × 10⁻⁵ |
| Ex. 6 | 7 | 5.1 × 10⁻⁵ |
| Comp. Ex. 2 | 8 | 2.4 × 10⁻⁵ |

The above results tell that the triphenylamine derivatives of the present invention have high carrier mobilities.

### Industrial Applicability:

The triphenylamine derivatives of the present invention have high carrier mobilities, excellent properties as a charge transporting agent, are very useful as a charge transporting agent when used for forming a photosensitive layer of an organic photosensitive material for electrophotography, and provide an organic photosensitive material for electrophotography having favorable properties such as a high sensitivity and a low residual potential.

## Claims

1. A triphenylamine derivative represented by the following general formula (1); 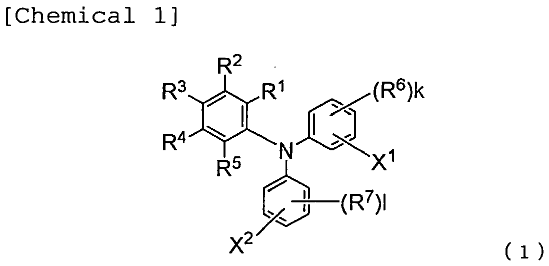 wherein,
k and l are respectively integers of 0 to 4,
R¹ to R⁵ may be the same or different, and are any groups selected from group consisting of hydrogen atom; deuterium atom; halogen atom; alkyl group having 1 to 6 carbon atoms; cycloalkyl group having 5 to 10 carbon atoms; alkenyl group having 2 to 6 carbon atoms; alkoxy group having 1 to 6 carbon atoms; cycloalkyloxy group having 5 to 10 carbon atoms; aromatic hydrocarbon group; aromatic heterocyclic ring group; condensed polycyclic aromatic group; and aryloxy group; or
among R¹ to R⁵, the two groups present at neighboring positions are bonded together to form a ring, and the other three groups are any groups selected from the above group;
R⁶ and R⁷ may be the same or different, and are the groups selected from the group consisting of deuterium atom; halogen atom; alkyl group having 1 to 6 carbon atoms; alkoxy group having 1 to 6 carbon atoms; aromatic hydrocarbon group; aromatic heterocyclic ring group; condensed polycyclic aromatic group; and
di-substituted amino group having, as a substituent, alkyl group having 1 to 6 carbon atoms, alkenyl group having 2 to 6 carbon atoms, aralkyl group, aromatic hydrocarbon group or aromatic heterocyclic ring group;,
if R⁶ and R⁷ are present in plural numbers, pluralities of R⁶ and R⁷ may be the same or different,
X¹ is a monovalent group represented by the following general formula (1a);
-CR⁸=CR⁹-CR¹⁰=CR¹¹R¹² (1a)
wherein,
R⁸ to R¹² may be the same or different and are hydrogen atoms, deuterium atoms, alkyl groups having 1 to 6 carbon atoms, alkoxy groups having 1 to 6 carbon atoms, aromatic hydrocarbon groups, aromatic heterocyclic ring groups or condensed polycyclic aromatic groups, wherein R¹¹ and R¹² together may form a ring and if R¹¹ is a hydrogen atom, deuterium atom or alkyl group, R¹² is an aromatic hydrocarbon group, aromatic heterocyclic ring group or condensed polycyclic aromatic group, and
X² is a monovalent group represented by the following general formula (1b);
- (CR¹³=CR¹⁴)ₙ-CR¹⁵=CR¹⁶R¹⁷ (1b)
wherein,
n is 0 or 1,
R¹³ to R¹⁷ may be the same or different and are hydrogen atoms, deuterium atoms, alkyl groups having 1 to 6 carbon atoms, alkoxy groups having 1 to 6 carbon atoms, aromatic hydrocarbon groups, aromatic heterocyclic ring groups or condensed polycyclic aromatic groups, wherein R¹⁶ and R¹⁷ together may form a ring and if R¹⁶ is a hydrogen atom, deuterium atom or alkyl group, R¹⁷ is an aromatic hydrocarbon group, aromatic heterocyclic ring group or condensed polycyclic aromatic group.

2. The triphenylamine derivative according to claim 1, which is represented by the following general formula (2); 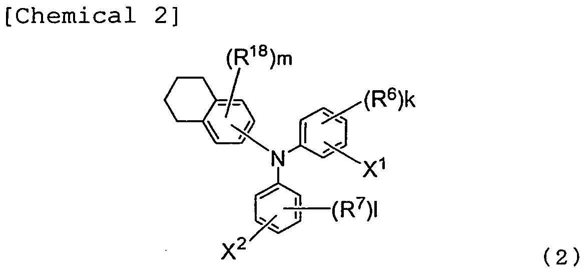 wherein,
k, l, R⁶, R⁷, X¹ and X² are respectively as defined in the above general formula (1),
m is an integer of 0 to 3,
R¹⁸ is any group selected from the group consisting of deuterium atom; halogen atom; alkyl group having 1 to 6 carbon atoms; cycloalkyl group having 5 to 10 carbon atoms; alkenyl group having 2 to 6 carbon atoms; alkoxy group having 1 to 6 carbon atoms; cycloalkyloxy group having 5 to 10 carbon atoms; aromatic hydrocarbon group; aromatic heterocyclic ring group; condensed polycyclic aromatic group; and aryloxy group;, and if there are a plurality of R¹⁸s, the plurality of R¹⁸s may be the same or different.

3. The triphenylamine derivative according to claim 2, which is represented by the following general formula (2-1); 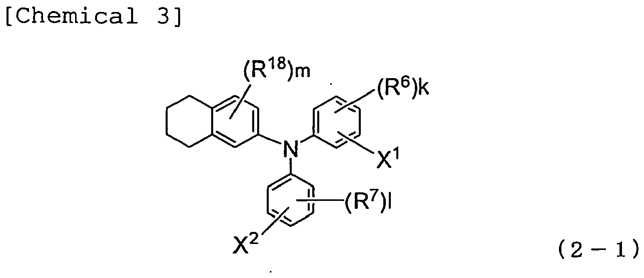 wherein,
k, l, m, R⁶, R⁷, R¹⁸, X¹ and X² are respectively as defined in the above general formula (2).

4. The triphenylamine derivative according to claim 3, which is represented by the following general formula (2-2); 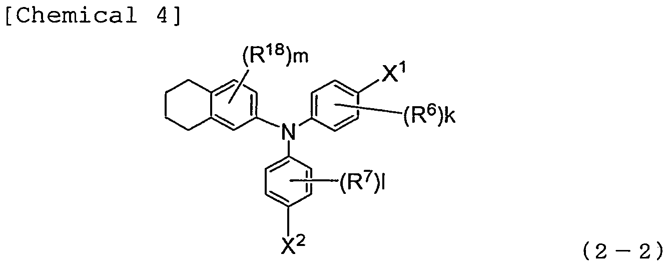 wherein,
k, l, m, R⁶, R⁷, R¹⁸, X¹ and X² are respectively as defined in the above general formula (2-1).

5. The triphenylamine derivative according to claim 4, which is represented by the following general formula (2-3); 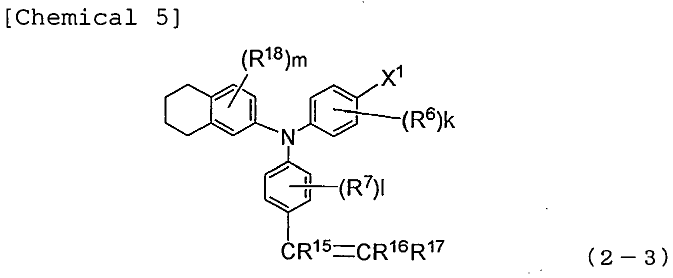 wherein,
k, l, m, R⁶, R⁷, R¹⁸ and X¹ are respectively as defined in the above general formula (2-2), and R¹⁵ to R¹⁷ are respectively as defined in the above general formula (1b).

6. The triphenylamine derivative according to claim 4, which is represented by the following general formula (2-3'); 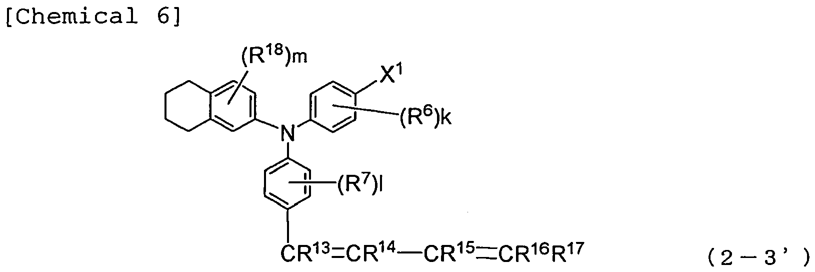 wherein,
k, l, m, R⁶, R⁷, R¹⁸ and X¹ are respectively as defined in the above general formula (2-2), and R¹³ to R¹⁷ are as defined in the above general formula (1b).

7. The triphenylamine derivative according to claim 1, which is represented by the following general formula (3); 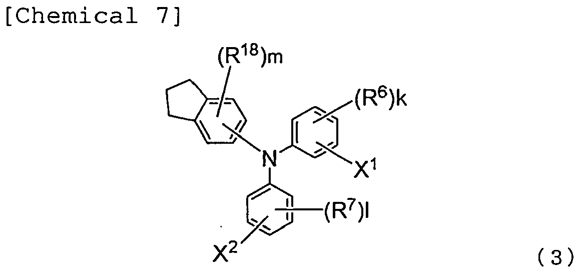 wherein,
k, l, R⁶, R⁷, X¹ and X² are respectively as defined in the above general formula (1),
m is an integer of 0 to 3,
R¹⁸ is any group selected from the group consisting of deuterium atom; halogen atom; alkyl group having 1 to 6 carbon atoms; cycloalkyl group having 5 to 10 carbon atoms; alkenyl group having 2 to 6 carbon atoms; alkoxy group having 1 to 6 carbon atoms; cycloalkyloxy group having 5 to 10 carbon atoms; aromatic hydrocarbon group; aromatic heterocyclic ring group; condensed polycyclic aromatic group; and aryloxy group;, and if there are a plurality of R¹⁸s, the plurality of R¹⁸s may be the same or different.

8. The triphenylamine derivative according to claim 7, which is represented by the following general formula (3-1); 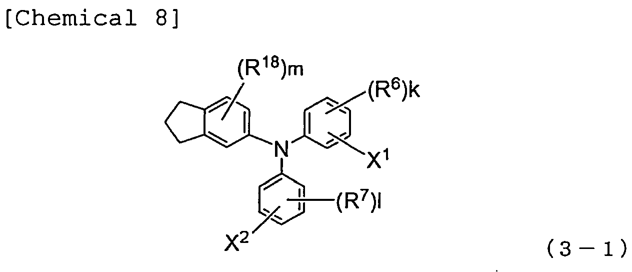 wherein,
k, l, m, R⁶, R⁷, R¹⁸, X¹ and X² are respectively as defined in the above general formula (3).

9. A charge transporting agent comprising the triphenylamine derivative of claim 1.

10. An organic photosensitive material for electrophotography having an organic photosensitive layer formed on an electrically conducting substrate, said organic photosensitive layer containing the triphenylamine derivative of claim 1 as a charge transporting agent.

11. The organic photosensitive material for electrophotography according to claim 10, wherein-said organic photosensitive layer is a lamination type photosensitive layer which comprises a charge generating layer in which a charge generating agent is dispersed in a resin binder, and a charge transporting layer in which the charge transporting agent is dispersed in a resin binder.

12. The organic photosensitive material for electrophotography according to claim 10, wherein said organic photosensitive layer is a single-layer type photosensitive layer in which the charge generating agent and the charge transporting agent are dispersed in a resin binder.
